# EUROPEAN PATENT APPLICATION

(11) **EP 4 349 343 A2**
(43) Date of publication of application: **10.04.2024**
(21) Application number: 23216025.9
(22) Date of filing: 30.11.2012
(51) Int. Cl.: A61K 31/713

(54) **EXPRESSION OF MIRNAS IN PLACENTAL TISSUE**

(30) Priority: 30.11.2011 EP 11191362; 05.04.2012 EP 12163414; 14.08.2012 EP 12180419
(62) Divisional of application: 16201258.7
(71) Applicant: Bullerdiek, Jörn, 28357 Bremen (DE)
(72) Inventor: Flor, Inga, 28209 Bremen (DE); Bullerdiek, Jörn, 28357 Bremen (DE)
(74) Representative: Witthoff Jaekel Steinecke Patentanwälte PartG mbB

(57) **Abstract**

Provided are human miRNAs associated with the generation of immunological tolerance during pregnancy as well as fragments, derivatives and variants thereof for use in immunomodulation. Said miRNAs may be used in diagnosis and treatment of disorders associated with a deregulated immune response, autoimmune disorders, pregnancy associated diseases, failure or problems of placentation and complications resulting from allo-transplantations. In addition, new pharmaceutical and diagnostic compositions for use in diagnosis and therapy of said disorders are described.

## Description

### Field of the invention

The present invention generally relates to binding molecules useful for immunomodulation, particularly human miRNAs as well as precursors, derivatives, variants and mimics thereof that are involved or can be used in the modulation of the immune system. In addition, the present invention relates to pharmaceutical and diagnostic compositions comprising such binding molecules, precursors and mimics thereof valuable both as a diagnostic tool to identify mutations in transcription units involved in modulation of the maternal immune system during pregnancy and also in strategies for treating disorders related to a misregulated or overactive immune system such as pregnancy-associated diseases, failure or problems of placentation, autoimmune diseases or in prevention or treatment of graft-versus-host reactions.

### BACKGROUND OF THE INVENTION

For successful pregnancy, modulation of the maternal immune system is necessary. It is known that the embryonic/foetal part of the placenta and in particular its trophoblast is able to produce factors that can prevent rejection of the embryo by interacting with the maternal immune system locally, i.e. within the decidua, as well as in the periphery. However, the immunomodulation has to work efficiently already during early pregnancy. In the first trimester of pregnancy, no less than 30-40% of the decidua consists of maternal immune cells the majority (about 70%) of which are NK cells but macrophages and T cells are known to occur in considerable percentages as well (Warning et al., 2011).

While some of these factors mediating immunomodulation are known, others still remain to be identified. The ability to modulate the maternal immune system is not confined to the trophoblast but foetal stromal cells are able as well to execute an efficient cross-talk with cells of the mother's immune system (Roelen et al., 2009). Experiments aimed at the identification of mechanisms responsible for the modulation of the maternal immune systems seem to indicate soluble factors as well as particles playing an important role in this process. Among the latter are exosomes, i.e. small membrane vesicles that can be released from a variety of cell types and contain a diverse cargo consisting of proteins, lipids as well as mRNAs and microRNAs (Valadi et al., 2007). During pregnancy, cells of the trophoblast can secrete exosomes which seem to suppress the maternal immune system (Southcombe et al., 2011). However, it is not known how this suppression is actually achieved and which factors exactly are involved therein. The identification of such factors would provide new means for therapeutic and diagnostic strategies within the field of pregnancy associated disorders or complications during pregnancy and might be useful as well in treatment of several diseases associated with a deregulated or overactive immune system.

This technical problem has been solved by the embodiments as characterized in the claims and described further below.

### SUMMARY OF THE INVENTION

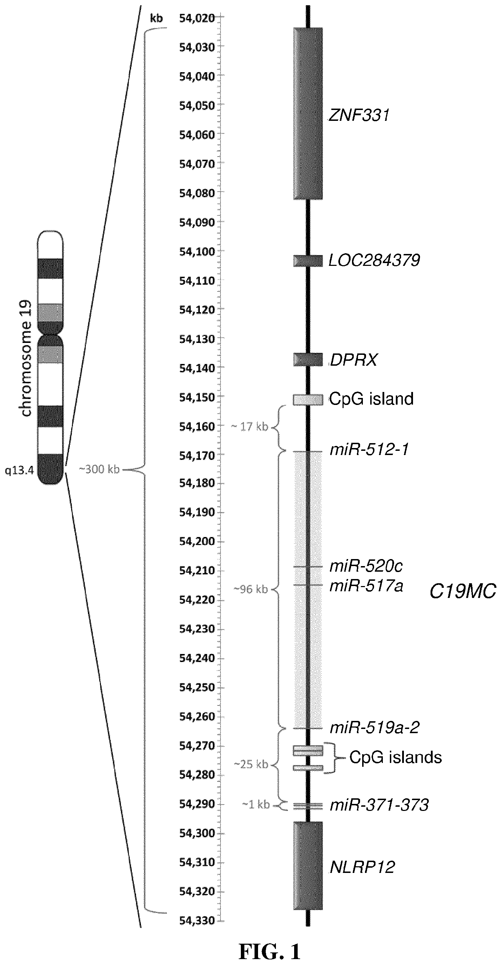

Herein, data is presented which surprisingly pinpoints an early function of the C19MC miRNAs in early pregnancy and in placental stromal cells. This data implicates that miRNAs of the C19MC as well as of the miR-371-3 cluster are important immunomodulators. The genes encoding both clusters have been assigned in close proximity to each other on the long arm of chromosome 19 (Fig. 1). In respect of the data provided herein, the present invention generally relates to the provision of miRNAs, their precursors of the C19MC as well as of the miR-371-3 cluster for use in immunomodulation. Further miRNAs sharing a common seed sequence AAGTGC and binding molecules capable of interfering with the gene expression of a target gene of these miRNA molecules are provided in this respect as well. Furthermore, the present invention provides nucleic acids, vectors and host cells comprising the nucleic acid sequence of the miRNA molecules as defined hereinabove and below, wherein the nucleic acid sequences are operably linked to regulatory sequences, e.g., promoters, enhancers, which are used for the induction and control of expression of the above mentioned miRNA molecules, their precursors or further binding molecules of the present invention in the host cells and/or in patients. In this respect, the present invention further provides pharmaceutical and diagnostic compositions useful in treatment or diagnosis of disorders generally linked to a deregulated immune system, as in case of autoimmune diseases, pregnancy-associated diseases, failure or problems of placentation or implantation, and treatment or prevention of rejection reactions of allografts (transplants) or due to the graft-versus-host reactions. Furthermore, the present invention also provides agents useful in treatment of cells attacked by autoimmune diseases or prevention or treatment of destruction of autologous tissue in case of autoimmune diseases, for example. Agents provided by the present invention and used in the above mentioned compositions are designed for different forms of administration, such as local or systemic administration. Other embodiments of the invention will be apparent from the description that follows and the items below, which are part of the preferred embodiments.
[1] A miRNA for use in immunomodulation, wherein the miRNA is selected from the miRNAs encoded by any one of the transcription units comprised in the C19MC cluster or in the miR-371-373 cluster.
[2] The microRNA of [1], wherein the microRNA is selected from the group consisting of: hsa-miR-498, hsa-miR-512-3p, hsa-miR-512-5p, hsa-miR-515-3p, hsa-miR-515-5p, hsa-miR-516a-3p, hsa-miR-516a-5p, hsa-miR-516b-3p, hsa-miR-516b-5p, hsa-miR-517-5p, hsa-miR-517a-3p, hsa-miR-517b-3p, hsa-miR-517c-3p, hsa-miR-518a-3p, hsa-miR-518a-5p, hsa-miR-518b, hsa-miR-518c-3p, hsa-miR-518c-5p, hsa-miR-518d-3p, hsa-miR-518d-5p, hsa-miR-518e-3p, hsa-miR-518e-5p, hsa-miR-518f-3p, hsa-miR-518f-5p, hsa-miR-519a-3p, hsa-miR-519a-5p, hsa-miR-519b-3p, hsa-miR-519b-5p, hsa-miR-519c-3p, hsa-miR-519c-5p, hsa-miR-519d, hsa-miR-519e-3p, hsa-miR-519e-5p, hsa-miR-520a-3p, hsa-miR-520a-5p, hsa-miR-520b, hsa-miR-520c-3p, hsa-miR-520c-5p, hsa-miR-520d-3p, hsa-miR-520d-5p, hsa-miR-520e, hsa-miR-520f, hsa-miR-520g, hsa-miR-520h, hsa-miR-521, hsa-miR-522-3p, hsa-miR-522-5p, hsa-miR-523-3p, hsa-miR-523-5p, hsa-miR-524-3p, hsa-miR-524-5p, hsa-miR-525-3p, hsa-miR-525-5p, hsa-miR-526a, hsa-miR-526b-3p, hsa-miR-526b-5p, hsa-miR-527, hsa-miR-1283, hsa-miR-1323, hsa-miR-371a-3p, hsa-miR-371a-5p, hsa-miR-371b-3p, hsa-miR-371b-5p, hsa-miR-372, hsa-miR-373-3p, hsa-miR-373-5p having the SEQ ID Nos.: 1 to 66 .
[3] The microRNA of [1] or [2] having common seed sequences with miRNAs encoded from the cluster C19MC or miR-371-373, wherein the microRNA:
   a.) is selected from the group consisting of hsa-miR-302a-3p, hsa-miR-302a-5p, hsa-miR-302b-3p, hsa-miR-302b-5p, hsa-miR-302c-3p, hsa-miR-302c-5p, hsa-miR-302d-3p, hsa-miR-302d-5p having the SEQ ID Nos: 67 to 78; and/or
   b.) is characterized by the consensus seed sequence AAGTGC.
[4] A miRNA precursor comprising the nucleic acid sequence of the miRNA of any one of [1] to [3] for use in immunomodulation.
[5] A binding molecule capable of interfering with the gene expression of a target gene of the miRNA molecule of any one of [1] to [4] for use in immunomodulation, wherein the binding molecule is selected from the group of molecules comprising synthetic miRNA mimics, RNA-molecules, antibodies, aptamers, spiegelmers for use in immunomodulation.
[6] A double-stranded nucleic acid comprising the nucleic acid sequence of the miRNA or miRNA precursor of any one of [1] to [4] or of the binding molecule of [5].
[7] A vector comprising the double-stranded nucleic acid of [6].
[8] A host cell comprising the vector of [7].
[9] The host cell according to [8], wherein the cell is a human cell, preferably wherein the cell is selected from the group of patient's autologous cells.
[10] A pharmaceutical composition or a diagnostic agent comprising as an agent at least one miRNA according to any one of [1] to [3], the miRNA precursor of [4], the binding molecule of [5], the double-stranded nucleic acid of [6], the vector of [7] and/or the host cell of [8] or [9].
[11] The pharmaceutical composition or diagnostic agent of [10], wherein the active agent is embedded in artificial exosomes.
[12] The miRNA of any one of [1] to [3], the miRNA precursor of [4], the binding molecule of [5] or the pharmaceutical composition of [10] or [11] for use in the treatment of an autoimmune disease.
[13] The use according to [12], wherein the autoimmune disease is selected from the group of diseases comprising Acute disseminated encephalomyelitis (ADEM), Alopecia areata, Ankylosing Spondylitis, Antiphospholipid syndrome (APS), Autoimmune cardiomyopathy, Autoimmune hemolytic anemia, Autoimmune hepatitis, Autoimmune inner ear disease, Autoimmune lymphoproliferative syndrome (ALPS), Autoimmune peripheral neuropathy, Autoimmune pancreatitis, Autoimmune polyendocrine syndrome, Autoimmune progesterone dermatitis, Autoimmune thrombocytopenic purpura, Autoimmune urticaria, Autoimmune uveitis, Celiac disease, Cold agglutinin disease, Crohns Disease, Dermatomyositis, Diabetes mellitus type 1, Endometriosis, Eosinophilic fasciitis, Gastrointestinal pemphigoid, Goodpasture's syndrome, Graves' disease, Guillain-Barré syndrome (GBS), Hashimoto's encephalopathy, Hashimoto's thyroiditis, Idiopathic thrombocytopenic purpura (Autoimmune thrombocytopenic purpura), Lupus erythematosus, Miller-Fisher syndrome (Guillain-Barre-Syndrome), Mixed Connective Tissue Disease, Myasthenia gravis, Pemphigus vulgaris, Pernicious anaemia, Polymyositis, Primary biliary cirrhosis, Psoriasis, Psoriatic arthritis, Relapsing polychondritis, Rheumatoid arthritis, Sjögren's syndrome, Temporal arteritis ("giant cell arteritis"), Transverse myelitis, Ulcerative colitis, Undifferentiated connective tissue disease (Mixed connective tissue disease), Vasculitis, Wegener's granulomatosis.
[14] The miRNA of any one of [1] to [3], the miRNA precursor of [4], the binding molecule of [5], the pharmaceutical composition of [10] or [11] or the diagnostic agent of [10] or [11] for use in treatment or diagnosis of a pregnancy-associated disease.
[15] The miRNA of any one of [1] to [3], the miRNA precursor of [4], the binding molecule of [5], the pharmaceutical composition of [10] or [11] or the diagnostic agent of [10] or [11] according to [14], wherein the pregnancy-associated disease is selected from the group of diseases consisting of eclampsia, pre-eclampsia, HELLP-syndrome and failure or problems of placentation or implantation.
[16] The miRNA of any one of [1] to [3], the miRNA precursor of [4], the binding molecule of [5], the pharmaceutical composition of [10] or [11] for use in prevention or treatment of rejection of allografts.
[17] The miRNA of any one of [1] to [3], the miRNA precursor of [4], the binding molecule of [5], the pharmaceutical composition of [10] or [11] for use in prevention or treatment of graft-versus-host reactions.
[18] The miRNA of any one of [1] to [3], the miRNA precursor of [4], the binding molecule of [5], the pharmaceutical composition of [10] or [11] or the diagnostic agent of [10], which are designed for local administration.
[19] The miRNA of any one of [1] to [3], the miRNA precursor of [4], the binding molecule of [5], the pharmaceutical composition of [10] or [11] or the diagnostic agent of [10], which are designed for systemic administration.
[20] The miRNA of any one of [1] to [3, the miRNA precursor of [4], the binding molecule of [5], the vector of [7], the pharmaceutical composition of [10] or [11], or the diagnostic agent of [10] or [11] for use in the ex vivo and/or in vivo treatment of allografts.
[21] The miRNA of any one of [1] to [3], the miRNA precursor of [4], the binding molecule of [5], the vector of [7], the pharmaceutical composition agent of [10] or [11], or the diagnostic agent of [10] or [11] for use in the ex vivo and/or in vivo treatment of autologous cells or tissues.
[22] The miRNA of any one of [1] to [3], the miRNA precursor of [4], the binding molecule of [5], the vector of [7], the pharmaceutical composition agent of [10] or [11], or the diagnostic agent of [10] or [11] for use in the ex vivo and/or in vivo treatment of cells or tissues attacked by autoimmune diseases.
[23] An antagonist directed against the miRNA of any one of [1] to [3] and/or against a miRNA precursor of [4] selected from the group of molecules comprising synthetic miRNA mimics, RNA-molecules, antibodies, aptamers, spiegelmers and small molecules for use in treatment of benign and malignant tumors selected from the group comprising tumors of the thyroid, breast cancer, colon cancer, lung cancer, ovarian cancer, germ cell tumors, hepatocellular cancer, leukaemia and lymphoma.
[24] A method for obtaining exosomes for use in immunomodulation comprising isolation and purification of exosomes from a supernatant of cell cultures of embryonic or fetal cells expressing miRNAs of the C19MC, the miR-371-373 and/or the miR302-367 cluster of any one of [1] to [3].
[25] The method of [24], wherein the cell cultures are selected from the group of cells comprising cells from the umbilical cord, the amniotic membrane, the placenta, and chorionic membrane.
[26] A method for obtaining exosomes for use in immunomodulation from a biological sample comprising the steps of setting up a cell culture from the biological sample, collecting the supernatant of the cell culture and isolating and purifying the exosomes thereof.
[27] The method of [26], wherein the biological sample comprises autologous cells, tissue sample or aspirate.
[28] The method of [26], wherein the biological sample comprises allologous cells, tissue sample or aspirate.
[29] A method for in vitro generation of exosomes comprising miRNAs, binding molecules and/or double stranded nucleic acids according to any one of [1] to [3], [5] and [6] and exosomes obtained according to any one of [24] to [28] for use in immunomodulation.
[30] Exosomes obtained according to the method of any one of [24] to [29] for use in treatment of an autoimmune disease.
[31] Exosomes obtained according to the method of any one of [24] to [29] for use in treatment of an autoimmune disease by a local administration.
[32] Exosomes of [31], wherein the exosomes are administered using joint injection, preferably intra-articular injection or intra-nasal application.
[33] Exosomes obtained according to the method of any one of [24] to [29] for use in treatment of an autoimmune disease by a systemic administration.
[34] Use of Azacytidine or other DNA demethylating agents for the treatment of cell cultures in the method of any one of [24] to [28] to enhance the ability of the cells to secrete exosomes.
[35] Azacytidine or other DNA demethylating agents for the use in treatment of tissues in vivo to enhance their ability to secrete exosomes enhanced for miRNAs of the C19MC, the miR-371-373 and/or the miR302-367 cluster of any one of [1] to [3].
[36] A method to diagnose the ability of an embryo to implant or of a sperm sample to fertilize comprising identification of at least one miRNA of the C19MC cluster and/or the miR-371-373 cluster of any one of [1] to [3] in cell culture medium of blastocysts or embryos or in seminal fluid, respectively.
[37] The method of [36], wherein a comparable or increased level of the at least one miRNA of the C19MC cluster and/or the miR-371-373 cluster compared to a control sample is indicative of a normal or increased ability of the embryo to implant or of a sperm sample to fertilize and a decreased level of the at least one miRNA compared to a control sample is indicative of a reduced ability of the embryo to implant or of a sperm sample to fertilize.
[38] The method of [36], wherein the presence of the at least one miRNA of the C19MC cluster and/or the miR-371-373 cluster is indicative of a normal or increased ability of the embryo to implant or of a sperm sample to fertilize and the absence of the at least one miRNA of said cluster is indicative of a reduced ability of the embryo to implant or of a sperm sample to fertilize.

### BRIEF DESCRIPTION OF THE DRAWINGS

- **Figure 1:**: Scheme of the chromosomal region 19q13 with the two microRNA clusters C19MC and miR-371-3
- **Figure 2:**: Relative expression of miRNA miR-520c-3p in 52 placenta tissues in relation to the week of gestation.
- **Figure 3:**: Microdissection of chorionic villi. From the chorionic villi (A) first the stromal core (B), then the trophoblast layer (C) was excised.
- **Figure 4:**: Relative expression of miRNAs: miR-371-3p, miR-372, miR-373, and miR-520c-3p in stromal and trophoblast cells.
- **Figure 5:**: Relative expression of miR-520c-3p and miR-517a-3p in decidua and trophoblast cells.
- **Figure 6:**: Scheme illustrating miRNAs targeting transcripts of genes acting as inhibitors of Fas - FasL induced apoptosis. Targets have been identified according to miRBase (Release 18).
- **Figure 7:**: Cell proliferation measured by BrdU assay in PBMCs cocultured with JEG-3 cells and bAMCs at 96h and 120h, respectively.
- **Figure 8:**: Cell proliferation measured by BrdU assay in PBMCs alone, in mixed lymphocyte reactions and stimulated with Con A at 96h and 120h, respectively.
- **Figure 9:**: Genomic location of BC280723.
- **Figure 10:**: Relative expression of miR-517a-3p in bovine amniotic membrane-derived cells transfected with a BAC vector encoding C19MC compared to mock transfected cells at 24h, 48h and 6d after transfection. For 24h and 6d the transfection was performed in duplicate.
- **Figure 11:**: (**A**) Relative expression of miR-520c-3p in HCT-116 and JEG-3 cells (for description of quantitative RT-PCR see Example 1). (**B**) Relative expression of c-FLIP in Jurkat cells treated with supernatants of JEG-3 cells and HCT-1 16 cells, respectively.

### DETAILED DESCRIPTION OF THE INVENTION

While the miR-371-373 (371-3) cluster contains only seven microRNAs, C19MC is the largest currently known human miRNA cluster at all with roughly 60 different miRNAs (Tab. 1). Members of C19MC have been reported to be abundantly expressed in the trophoblast but not in the stromal part of the placenta and to have an increased expression during the pregnancy (Luo et al., 2009). Concerning the expression of the miR371-373 cluster in the placenta, no details are known yet.

However, microRNAs of both clusters have not been discussed as candidates that can modulate the maternal immune system yet. Strong arguments speaking against their involvement in maternal immunomodulation come from the existing literature. First, the expression of members of the C19MC cluster seems to be confined to the trophoblast (Luo et al., 2009) but placenta stromal cells have been described as having immunosuppressive functions as well (Roelen et al., 2009). Secondly, the increase of the expression of miRNA members of the C19MC cluster (Luo et al., 2009) leads to the conclusion that these miRNAs have functions rather related to late stages of pregnancy.

However, data presented within the present invention pinpoints in contrast to the above an early function of the C19MC miRNAs in early pregnancy and in placental stromal cells (see Examples 1 to 4). In respect of these experimental results described in detail further below, miRNAs of the C19MC as well as of the miR-371-3 cluster are provided herewith as important modulators of the maternal immune system.

Due to the ability of a single miRNA to interact with more than one target as well as due to the variety of different miRNAs in the miR-371-3 and - to an even much greater extent - in the C19MC cluster, the immunomodulatory functions of the miRNAs can be based on a variety of mechanisms. As an example only, without wishing to be bound by theory, pro-apoptotic functions have been depicted in Example 8 showing that members of the C19MC cluster interact with different mRNAs antagonizing Fas-FasL induced apoptosis. However, other relevant mechanisms including the induction of cellular senescence as well as reversible cell cycle arrest and anergy can be affected by miRNAs as well. Therefore, as to the therapeutic implications of these findings, in one embodiment of the present invention single miRNAs of both clusters described herein, the miRNAs of every single cluster as well as their different combinations are used to meet the intended therapeutic purpose.

**Table 1: MicroRNAs of the C19MC and the miR371-3 cluster assigned to chromosomal band 19q13 (cf. Fig. 1). ID Numbers according to entries in miRBase Release 18 (November 2011); see also Bentwich et al., Nat Genet. 37 (2005), 766-770 and Landgraf et al., Cell 129 (2007), 1401-1414. The sequences of the precursors of the miRNAs enlisted in the table can be obtained from the miRBase entries of the respective miRNAs.**

| miRNA ID according to miRBase | sequence | Cluster |
|---|---|---|
| hsa-miR-498 | UUUCAAGCCAGGGGGCGUUUUUC SEQ ID NO.: 1 | C19MC |
| hsa-miR-512-3p | AAGUGCUGUCAUAGCUGAGGUC SEQ ID NO.: 2 | C19MC |
| hsa-miR-512-5p | CACUCAGCCUUGAGGGCACUUUC SEQ ID NO.: 3 | C19MC |
| hsa-miR-515-3p | GAGUGCCUUCUUUUGGAGCGUU SEQ ID NO.: 4 | C19MC |
| hsa-miR-515-5p | UUCUCCAAAAGAAAGCACUUUCUG SEQ ID NO.: 5 | C19MC |
| hsa-miR-516a-3p | UGCUUCCUUUCAGAGGGU SEQ ID NO.: 6 | C19MC |
| hsa-miR-516a-5p | UUCUCGAGGAAAGAAGCACUUUC SEQ ID NO.: 7 | C19MC |
| hsa-miR-516b-3p | UGCUUCCUUUCAGAGGGU SEQ ID NO.: 8 | C19MC |
| hsa-miR-516b-5p | AUCUGGAGGUAAGAAGCACUUU SEQ ID NO.: 9 | C19MC |
| hsa-miR-517-5p | CCUCUAGAUGGAAGCACUGUCU SEQ ID NO.: 10 | C19MC |
| hsa-miR-517a-3p | AUCGUGCAUCCCUUUAGAGUGU SEQ ID NO.: 11 | C19MC |
| hsa-miR-517b-3p | AUCGUGCAUCCCUUUAGAGUGU SEQ ID NO.: 12 | C19MC |
| hsa-miR-517c-3p | AUCGUGCAUCCUUUUAGAGUGU SEQ ID NO.: 13 | C19MC |
| hsa-miR-518a-3p | GAAAGCGCUUCCCUUUGCUGGA SEQ ID NO.: 14 | C19MC |
| hsa-miR-518a-5p | CUGCAAAGGGAAGCCCUUUC SEQ ID NO.: 15 | C19MC |
| hsa-miR-518b | CAAAGCGCUCCCCUUUAGAGGU SEQ ID NO.: 16 | C19MC |
| hsa-miR-518c-3p | CAAAGCGCUUCUCUUUAGAGUGU SEQ ID NO.: 17 | C19MC |
| hsa-miR-518c-5p | UCUCUGGAGGGAAGCACUUUCUG SEQ ID NO.: 18 | C19MC |
| hsa-miR-518d-3p | CAAAGCGCUUCCCUUUGGAGC SEQ ID NO.: 19 | C19MC |
| hsa-miR-518d-5p | CUCUAGAGGGAAGCACUUUCUG SEQ ID NO.: 20 | C19MC |
| hsa-miR-518e-3p | AAAGCGCUUCCCUUCAGAGUG SEQ ID NO.: 21 | C19MC |
| hsa-miR-518e-5p | CUCUAGAGGGAAGCGCUUUCUG SEQ ID NO.: 22 | C19MC |
| hsa-miR-518f-3p | GAAAGCGCUUCUCUUUAGAGG SEQ ID NO.: 23 | C19MC |
| hsa-miR-518f-5p | CUCUAGAGGGAAGCACUUUCUG SEQ ID NO.: 24 | C19MC |
| hsa-miR-519a-3p | AAAGUGCAUCCUUUUAGAGUGU SEQ ID NO.: 25 | C19MC |
| hsa-miR-519a-5p | CUCUAGAGGGAAGCGCUUUCUG SEQ ID NO.: 26 | C19MC |
| hsa-miR-519b-3p | AAAGUGCAUCCUUUUAGAGGUU SEQ ID NO.: 27 | C19MC |
| hsa-miR-519b-5p | CUCUAGAGGGAAGCGCUUUCUG SEQ ID NO.: 28 | C19MC |
| hsa-miR-519c-3p | AAAGUGCAUCUUUUUAGAGGAU SEQ ID NO.: 29 | C19MC |
| hsa-miR-519c-5p | CUCUAGAGGGAAGCGCUUUCUG SEQ ID NO.: 30 | C19MC |
| hsa-miR-519d | CAAAGUGCCUCCCUUUAGAGUG SEQ ID NO.: 31 | C19MC |
| hsa-miR-519e-3p | AAGUGCCUCCUUUUAGAGUGUU SEQ ID NO.: 32 | C19MC |
| hsa-miR-519e-5p | UUCUCCAAAAGGGAGCACUUUC SEQ ID NO.: 33 | C19MC |
| hsa-miR-520a-3p | AAAGUGCUUCCCUUUGGACUGU SEQ ID NO.: 34 | C19MC |
| hsa-miR-520a-5p | CUCCAGAGGGAAGUACUUUCU SEQ ID NO.: 35 | C19MC |
| hsa-miR-520b | AAAGUGCUUCCUUUUAGAGGG SEQ ID NO.: 36 | C19MC |
| hsa-miR-520c-3p | AAAGUGCUUCCUUUUAGAGGGU SEQ ID NO.: 37 | C19MC |
| hsa-miR-520c-5p | CUCUAGAGGGAAGCACUUUCUG SEQ ID NO.: 38 | C19MC |
| hsa-miR-520d-3p | AAAGUGCUUCUCUUUGGUGGGU SEQ ID NO.: 39 | C19MC |
| hsa-miR-520d-5p | CUACAAAGGGAAGCCCUUUC SEQ ID NO.: 40 | C19MC |
| hsa-miR-520e | AAAGUGCUUCCUUUUUGAGGG SEQ ID NO.: 41 | C19MC |
| hsa-miR-520f | AAGUGCUUCCUUUUAGAGGGUU SEQ ID NO.: 42 | C19MC |
| hsa-miR-520g | ACAAAGUGCUUCCCUUUAGAGUGU SEQ ID NO.: 43 | C19MC |
| hsa-miR-520h | ACAAAGUGCUUCCCUUUAGAGU SEQ ID NO.: 44 | C19MC |
| hsa-miR-521 | AACGCACUUCCCUUUAGAGUGU SEQ ID NO.: 45 | C19MC |
| hsa-miR-522-3p | AAAAUGGUUCCCUUUAGAGUGU SEQ ID NO.: 46 | C19MC |
| hsa-miR-522-5p | CUCUAGAGGGAAGCGCUUUCUG SEQ ID NO.: 47 | C19MC |
| hsa-miR-523-3p | GAACGCGCUUCCCUAUAGAGGGU SEQ ID NO.: 48 | C19MC |
| hsa-miR-523-5p | CUCUAGAGGGAAGCGCUUUCUG SEQ ID NO.: 49 | C19MC |
| hsa-miR-524-3p | GAAGGCGCUUCCCUUUGGAGU SEQ ID NO.: 50 | C19MC |
| hsa-miR-524-5p | CUACAAAGGGAAGCACUUUCUC SEQ ID NO.: 51 | C19MC |
| hsa-miR-525-3p | GAAGGCGCUUCCCUUUAGAGCG SEQ ID NO.: 52 | C19MC |
| hsa-miR-525-5p | CUCCAGAGGGAUGCACUUUCU SEQ ID NO.: 53 | C19MC |
| hsa-miR-526a | CUCUAGAGGGAAGCACUUUCUG SEQ ID NO.: 54 | C19MC |
| hsa-miR-526b-3p | GAAAGUGCUUCCUUUUAGAGGC SEQ ID NO.: 55 | C19MC |
| hsa-miR-526b-5p | CUCUUGAGGGAAGCACUUUCUGU SEQ ID NO.: 56 | C19MC |
| hsa-miR-527 | CUGCAAAGGGAAGCCCUUUC SEQ ID NO.: 57 | C19MC |
| hsa-miR-1283 | UCUACAAAGGAAAGCGCUUUCU SEQ ID NO.: 58 | C19MC |
| hsa-miR-1323 | UCAAAACUGAGGGGCAUUUUCU SEQ ID NO.: 59 | C19MC |
| hsa-miR-371a-3p | AAGUGCCGCCAUCUUUUGAGUGU SEQ ID NO.: 60 | miR-371-3 |
| hsa-miR-371a-5p | ACUCAAACUGUGGGGGCACU SEQ ID NO.: 61 | miR-371-3 |
| hsa-miR-371b-3p | AAGUGCCCCCACAGUUUGAGUGC SEQ ID NO.: 62 | miR-371-3 |
| hsa-miR-371b-5p | ACUCAAAAGAUGGCGGCACUUU SEQ ID NO.: 63 | miR-371-3 |
| hsa-miR-372 | AAAGUGCUGCGACAUUUGAGCGU SEQ ID NO.: 64 | miR-371-3 |
| hsa-miR-373-3p | GAAGUGCUUCGAUUUUGGGGUGU SEQ ID NO.: 65 | miR-371-3 |
| hsa-miR-373-5p | ACUCAAAAUGGGGGCGCUUUCC SEQ ID NO.: 66 | miR-371-3 |

On the basis of this experimental data, the present invention generally relates to a miRNA for use in immunomodulation, wherein the miRNA is selected from the miRNAs encoded by any one of the transcription units comprised in the C19MC cluster or in the miR-371-373 cluster as enlisted in Table 1 above.

In one embodiment, the present invention the above mentioned microRNA is selected from the group consisting of: hsa-miR-498, hsa-miR-512-3p, hsa-miR-512-5p, hsa-miR-515-3p, hsa-miR-515-5p, hsa-miR-516a-3p, hsa-miR-516a-5p, hsa-miR-516b-3p, hsa-miR-516b-5p, hsa-miR-517-5p, hsa-miR-517a-3p, hsa-miR-517b-3p, hsa-miR-517c-3p, hsa-miR-518a-3p, hsa-miR-518a-5p, hsa-miR-518b, hsa-miR-518c-3p, hsa-miR-518c-5p, hsa-miR-518d-3p, hsa-miR-518d-5p, hsa-miR-518e-3p, hsa-miR-518e-5p, hsa-miR-518f-3p, hsa-miR-518f-5p, hsa-miR-519a-3p, hsa-miR-519a-5p, hsa-miR-519b-3p, hsa-miR-519b-5p, hsa-miR-519c-3p, hsa-miR-519c-5p, hsa-miR-519d, hsa-miR-519e-3p, hsa-miR-519e-5p, hsa-miR-520a-3p, hsa-miR-520a-5p, hsa-miR-520b, hsa-miR-520c-3p, hsa-miR-520c-5p, hsa-miR-520d-3p, hsa-miR-520d-5p, hsa-miR-520e, hsa-miR-520f, hsa-miR-520g, hsa-miR-520h, hsa-miR-521, hsa-miR-522-3p, hsa-miR-522-5p, hsa-miR-523-3p, hsa-miR-523-5p, hsa-miR-524-3p, hsa-miR-524-5p, hsa-miR-525-3p, hsa-miR-525-5p, hsa-miR-526a, hsa-miR-526b-3p, hsa-miR-526b-5p, hsa-miR-527, hsa-miR-1283, hsa-miR-1323, hsa-miR-371a-3p, hsa-miR-371a-5p, hsa-miR-371b-3p, hsa-miR-371b-5p, hsa-miR-372, hsa-miR-373-3p, hsa-miR-373-5p having the SEQ ID Nos.: 1 to 66 .

The seed sequence is essential for the binding of the miRNA to the mRNA. The "seed sequence" or "seed region", as referred to in the present invention, is a conserved heptametrical sequence which is mostly situated at positions 2-7 from the miRNA 5'-end. Even though base pairing of miRNA and its target mRNA does not match perfect, the "seed sequence" has to be perfectly complementary. Therefore, the present invention further relates to miRNAs having common seed sequences with miRNAs encoded from the cluster C19MC or miR-371-3, wherein the microRNA:
a.) is selected from the group consisting of hsa-miR-302a-3p, hsa-miR-302a-5p, hsa-miR-302b-3p, hsa-miR-302b-5p, hsa-miR-302c-3p, hsa-miR-302c-5p, hsa-miR-302d-3p, hsa-miR-302d-5p having the SEQ ID Nos: 67 to 78; and/or
b.) is characterized by the consensus seed sequence AAGTGC.
   miRNAs and their precursors of the above mentioned group consisting of hsa-miR-302a-3p, hsa-miR-302a-5p, hsa-miR-302b-3p, hsa-miR-302b-5p, hsa-miR-302c-3p, hsa-miR-302c-5p, hsa-miR-302d-3p, hsa-miR-302d-5p are encoded from the cluster miR302-367, their sequences and accession numbers are enlisted in Table 2 below.

**Table 2.: miRNAs and their precursors of cluster miR302-367 with the seed sequence AAGTGC common with the seed sequences of miRNAs encoded from the cluster C19MC or miR-371-3; see also Landgraf et al., Cell 129 (2007), 1401-1414 and Suh et al., Dev Biol. 270 (2004), 488-498**

| Precursor miRNA Sequence SEQ ID No. | Mature miRNA sequence SEQ ID No. | Cluster |
|---|---|---|
| miRBase Accession No. | miRBase Accession No. | |
| miRBase ID No. | miRBase ID No. | |
| CCACCACUUAAACGUGGAUG UACUUGCUUUGAAACUAAAG AAGUAAGUGCUUCCAUGUUU UGGUGAUGG | UAAGUGCUUCCAUGUUUUGGUGA SEQ ID NO.: 68 | miR302-367 |
| | MIMAT0000684 | |
| | hsa-miR-302a-3p | |
| SEQ ID NO.: 67 | ACUUAAACGUGGAUGUACUUGCU SEQ ID NO.: 69 | miR302-367 |
| MI0000738 | | |
| hsa-mir-302a | MIMAT0000683 | |
| | hsa-miR-302a-5p | |
| GCUCCCUUCAACUUUAACAU GGAAGUGCUUUCUGUGACUU UAAAAGUAAGUGCUUCCAUG UUUUAGUAGGAGU | UAAGUGCUUCCAUGUUUUAGUAG SEQ ID NO.: 71 | miR302-367 |
| | MIMAT0000715 | |
| SEQ ID NO.: 70 | hsa-miR-302b-3p | |
| MI0000772 | ACUUUAACAUGGAAGUGCUUUC SEQ ID NO.: 72 | miR302-367 |
| hsa-miR-302b | | |
| | MIMAT0000714 | |
| | hsa-miR-302b-5p | |
| CCUUUGCUUUAACAUGGGGG UACCUGCUGUGUGAAACAAA AGUAAGUGCUUCCAUGUUUC AGUGGAGG | UAAGUGCUUCCAUGUUUCAGUGG SEQ ID NO.: 74 | miR302-367 |
| | MIMAT0000717 | |
| | hsa-miR-302c-3p | |
| SEQ ID NO.: 73 | UUUAACAUGGGGGUACCUGCUG SEQ ID NO.: 75 | miR302-367 |
| MI0000773 | | |
| hsa-miR-302c | MIMAT0000716 | |
| | hsa-miR-302c-5p | |
| CCUCUACUUUAACAUGGAGG CACUUGCUGUGACAUGACAA AAAUAAGUGCUUCCAUGUUU GAGUGUGG | UAAGUGCUUCCAUGUUUGAGUGU SEQ ID NO.: 77 | miR302-367 |
| | MIMAT0000718 | |
| | hsa-miR-302d-3p | |
| SEQ ID NO.: 76 | ACUUUAACAUGGAGGCACUUGC SEQ ID NO.: 78 | miR302-367 |
| MI0000774 | | |
| hsa-miR-302d | MIMAT0004685 | |
| | hsa-miR-302d-5p | |

As explained in more detail further below, miRNAs are produced in the cell from longer primary transcripts, precursor RNA molecules, which may comprise the sequence of several mature miRNAs (Kim, Nature Rev. Mol. Cell Biol. 6 (2005), 376-385). Therefore, in one embodiment the present invention provides a miRNA precursor comprising the nucleic acid sequence of the miRNA as defined hereinbefore for use in immunomodulation. The sequences of the respective miRNA precursors of the miRNAs indicated in Tables 1 and 2 can, if not indicated already herein, be obtained from the respective entries in the miRBase.

miRNAs regulate gene expression by binding to mRNA of target genes. It is known in the art that this region may be targeted as well by other binding molecules, to achieve the regulatory effect induced in the normal case by the miRNA. Therefore, in one embodiment the present invention relates to a binding molecule capable of interfering with the gene expression of a target gene of the miRNA molecule as defined hereinbefore for use in immunomodulation, wherein the binding molecule is selected from the group of molecules comprising synthetic miRNA mimics, RNA-molecules, antibodies, aptamers, spiegelmers for use in immunomodulation.

In one embodiment the present invention further provides a double-stranded nucleic acid comprising the nucleic acid sequence of the miRNA, or miRNA precursor or of the binding molecule as defined hereinbefore.

Furthermore, in one embodiment the present invention provides one or more of above defined double-stranded nucleic acid of a sequence as defined hereinabove, wherein the miRNA, precursor miRNA and/or other binding molecules of the present invention encoding nucleotide sequences are operatively linked to at least one expression control sequence. Examples of such expression control sequences are promoter, operator, enhancer, silencer sequences, transcription terminators, polyadenylation sites and other nucleic acid sequences known in the art which may be used for the expression of miRNA, precursor miRNA and/or other binding molecules of the present invention.

Said expression control sequences may enhance or downregulate the expression levels of the miRNA encoding nucleotide sequences operatively linked to. One or several expression control sequences may be used in combination with each other and/or in combination with one or more of the miRNA, precursor miRNA and/or other binding molecules of the present invention encoding double-stranded nucleic acids (nucleotide sequences) as defined in the present invention depending on the cell type (e.g., prokaryotes or eukaryotes) or organism used for the expression of miRNA, precursor miRNA and/or other binding molecules of the present invention encoding nucleotide sequences. The expression regulatory sequences may be chosen as well in respect of the time (*i.e.* developmental stage), cell type and/or general circumstances, *e.g*., the presence and/or absence of one or more specific substances, wherein the miRNA and/or miRNA precursor molecule encoded by the nucleotide sequences as defined hereinabove are expressed when said regulatory sequences operably linked to the polypeptide and/or peptide encoding nucleotide sequences permit their expression because one or more of the mentioned conditions are met or not expressed, when the circumstances permitting expression are not met.

The expression control sequences used may originate from the same organism as the miRNA, precursor miRNA and/or other binding molecules of the present invention encoding nucleotide sequences of the present invention as defined hereinabove or they may be foreign, *i.e*. originate from another organism in the meaning of different taxonomy or phylogeny. In one embodiment the present invention provides a nucleic acid molecule comprising the polypeptide and/or peptide encoding nucleotide sequences, wherein at least one expression control sequence is foreign to the polypeptide and/or peptide encoding nucleotide sequences.

The polynucleotide as employed in accordance with this invention and encoding the above described miRNA, precursor miRNA and/or other binding molecules of the present invention may be, *e.g*., DNA, cDNA, RNA or synthetically produced DNA or RNA or a recombinantly produced chimeric nucleic acid molecule comprising any of those polynucleotides either alone or in combination. Preferably, the polynucleotides are operatively linked to expression control sequences allowing expression in prokaryotic or eukaryotic cells. Expression of said polynucleotide comprises transcription of the polynucleotide into a translatable mRNA. Details describing the expression of the polynucleotides of the present invention will be described further below in this description.

In this respect, the present invention provides in one embodiment a vector comprising the double-stranded nucleic acid as defined hereinabove. Furthermore, in one embodiment of the present invention a host cell comprising the vector is provided. In one embodiment of the present invention the host cell as defined hereinabove and below is a human cell, preferably wherein the cell is selected from the group of patient's autologous cells.

In another embodiment, the present invention relates to a pharmaceutical composition or a diagnostic agent comprising as an agent at least one miRNA, the miRNA precursor, the binding molecule, the double-stranded nucleic acid, the vector and/or the host cell as defined hereinabove and below.

In one embodiment of the pharmaceutical composition or diagnostic agent, the active agent is embedded in artificial exosomes (see also Example 4).

In one embodiment the present invention provides the miRNA, the miRNA precursor, the binding molecule or the pharmaceutical composition as defined hereinabove for use in the treatment of an autoimmune disease.

In one embodiment the present invention relates to the use as defined hereinabove, wherein the autoimmune disease is selected from the group of diseases comprising Acute disseminated encephalomyelitis (ADEM), Alopecia areata, Ankylosing Spondylitis, Antiphospholipid syndrome (APS), Autoimmune cardiomyopathy, Autoimmune hemolytic anemia, Autoimmune hepatitis, Autoimmune inner ear disease, Autoimmune lymphoproliferative syndrome (ALPS), Autoimmune peripheral neuropathy, Autoimmune pancreatitis, Autoimmune polyendocrine syndrome, Autoimmune progesterone dermatitis, Autoimmune thrombocytopenic purpura, Autoimmune urticaria, Autoimmune uveitis, Celiac disease, Cold agglutinin disease, Crohns Disease, Dermatomyositis, Diabetes mellitus type 1, Endometriosis, Eosinophilic fasciitis, Gastrointestinal pemphigoid, Goodpasture's syndrome, Graves' disease, Guillain-Barré syndrome (GBS), Hashimoto's encephalopathy, Hashimoto's thyroiditis, Idiopathic thrombocytopenic purpura (Autoimmune thrombocytopenic purpura), Lupus erythematosus, Miller-Fisher syndrome (Guillain-Barre-Syndrome), Mixed Connective Tissue Disease, Myasthenia gravis, Pemphigus vulgaris, Pernicious anaemia, Polymyositis, Primary biliary cirrhosis, Psoriasis, Psoriatic arthritis, Relapsing polychondritis, Rheumatoid arthritis, Sjögren's syndrome, Temporal arteritis ("giant cell arteritis"), Transverse myelitis, Ulcerative colitis, Undifferentiated connective tissue disease (Mixed connective tissue disease), Vasculitis, Wegener's granulomatosis.

In another embodiment the present invention provides the miRNA, the miRNA precursor, the binding molecule, the pharmaceutical composition or the diagnostic agent as defined hereinabove for use in treatment or diagnosis of a pregnancy-associated disease.

In one embodiment of the use in treatment or diagnosis a pregnancy-associated disease as defined hereinabove, the pregnancy-associated disease is selected from the group of diseases consisting of eclampsia, pre-eclampsia, HELLP-syndrome and failure or problems of placentation or implantation. Eclampsia, pre-eclampsia and the HELLP-syndrome are all known in the art to be associated with placental dysfunction; see, e.g., Benedetto et al., Adv Clin Chem 53 (2011), 85-104.

"Allografts" are herein defined as a transplant of an organ, tissue or cells from one individual to another of the same species with a different genotype. In case of such transplantation, rejection reactions can be observed very often due to the recognition of the transplant by the host's immune system as foreign and mounting an immune reaction against it. Therefore, an immune suppressive treatment is required following the transplantation of the allograft for reduction of acute rejection. In this respect, in one embodiment the present invention provides the miRNA, the miRNA precursor, the binding molecule, the pharmaceutical composition as defined hereinabove for use in prevention or treatment of rejection of allografts. Since both, prevention and/or treatment of rejection of allografts can be performed using the molecules or compositions of the present invention, these molecules/compositions may be used in order to obviate and/or to alleviate, before and/or after the actual transplantation, or concerning the place of treatment within and as well outside of a living organism, e.g., a human patient. Therefore, in one embodiment the present invention provides the miRNA, the miRNA precursor, the binding molecule, the vector, the pharmaceutical composition, or the diagnostic agent as defined hereinabove and below for use in the ex vivo and/or in vivo treatment of allografts.

A specific form of rejection reactions, "graft-versus-host reactions", occurs when immunologically competent cells are transplanted between patients of different genotype, e.g., as in the case of bone marrow, stem cell transplantations or blood transfusions. If the host possesses important alloantigens that are lacking in the donor graft, the host appears foreign to the graft and can therefore stimulate it antigenically. Therefore, in one embodiment the present invention also provides the miRNA, the miRNA precursor, the binding molecule, the pharmaceutical composition of claim as defined hereinabove for use in prevention or treatment of graft-versus-host reactions.

In another embodiment the present invention provides the miRNA, the miRNA precursor, the binding molecule, the vector, the pharmaceutical composition, or the diagnostic agent as defined hereinabove and below for use in the ex vivo and/or in vivo treatment of cells or tissues attacked by autoimmune diseases. Furthermore, in one embodiment the present invention also provides the miRNA, the miRNA precursor, the binding molecule, the vector, the pharmaceutical composition, or the diagnostic agent as defined hereinabove and below for use in the ex vivo and/or in vivo treatment of autologous cells or tissues.

The present invention further relates to methods, molecules and compositions useful in modulating the immune system by increasing the immune response and/or reducing the immune tolerance of an organism against defined cells and/or tissues. In particular, the present invention also relates to new methods, molecules and compositions for cancer therapy. Genetic and biochemical characterization of tumor antigens has led to the discovery that most cancer patients mount some form of immune response to developing neoplasms. However, the formation and progression of clinically evident disease implies that endogenous reactions are typically ineffectual. Several features of tumor cell biology contribute to the relatively weak anti-tumor response. First, since tumor cells are derived from normal, non-foreign cells, the immune system may not recognize the tumor cell as dangerous or foreign. Second, tumor cells tend to express a reduced complement of the receptors and molecules that the body relies upon to activate immune responses. The present invention relates to a third mechanism, i.e. the use of particular miRNAs by the cancer cells to protect themselves.

There are several approaches to stimulating and potentiating anti-tumor immunity, such as cancer vaccines against specific tumor cell antigens or whole tumor cells, monoclonal antibodies, recombinant cytokines and adaptive cellular infusions (Pandolfi et al., 2011), new methods in this respect are still required however. Data presented within the present invention, pinpoint towards an important role of particular miRNAs and miRNA precursor molecules as defined hereinabove in modulating the immune system indicate their potential as a target in tumor therapy as well.

Therefore, in one embodiment the present invention provides an antagonist directed against the miRNAs and/or against a miRNA precursor as defined hereinabove selected from the group of molecules comprising synthetic miRNA mimics, RNA-molecules, antibodies, aptamers, spiegelmers and small molecules for use in treatment of benign and malignant tumors selected from the group comprising tumors of the thyroid, breast cancer, colon cancer, lung cancer, ovarian cancer, germ cell tumors, hepatocellular cancer, leukaemia and lymphoma.

The miRNA, the miRNA precursor, the binding molecule, the pharmaceutical composition or the diagnostic agent as defined hereinabove may be applied in different forms as known in the art and may be designed for different forms of administration. In one embodiment of the present invention the miRNA, the miRNA precursor, the binding molecule, the pharmaceutical composition or the diagnostic agent as defined hereinabove are designed for local administration. In another embodiment however, they are designed for systemic administration.

The present invention further relates to novel methods and materials for obtaining, generating, isolating and/or purifying exosomes from biological samples such as cells, tissue, blood, aspirate, amniotic fluid and supernatants of, e.g., cells or cell cultures. General methods for isolation and purification of exosomes and their content such as miRNAs are known in the art. In addition, analogously or alternatively to the methods mentioned in section "Exosomes" further below and described in WO99/03499, WO00/44389 and WO97/05900, for example, exosomes may be isolated from biological samples by ultracentrifugation and their content, i.e., miRNAs can be purified with the TRIZOL reagent (Invitrogen, Carlsbad, CA) according to the protocol provided by the manufacturer and identified after reverse transcription to cDNA by PCR using specific miRNA primers as described in the subsections "Exosome isolation" and "RNA Extraction and Reverse Transription" on pages 4 to 5 of Gallo et al. (2012), PLoS One.;7(3):e30679, the disclosure content of which is hereby incorporated by reference. miRNA quantification can be performed as described in the "miRNA quantification" subsection on page 5 of Gallo et al. (2012), the disclosure content of which is hereby incorporated by reference. Alternatively, miRNAs can be isolated by Exosome RNA Isolation Kits offered by several manufacturers, such as Norgen Biotek Corp (Thorold, CANADA), e.g., Norgen's Urine Exosome RNA Isolation Kit (Norgen; Cat. No. 47200) for isolation and enrichment of exosomes from urine and tissue culture media, or Plasma/Serum Exosome RNA Isolation Kit (Norgen; Cat. No. 49200) for isolation and enrichment of exosomal RNA from of plasma, serum and ascitic fluid in accordance to the manual of the supplier. Furthermore, exosomes can be isolated instead of ultracentrifugation by the use of further kits, such as ExoQuick-TC^{™} from Gentaur (Kampenhout, Belgium) in accordance to the manual of the supplier.

These enriched samples can be used to determine the presence or absence of particular types of exosomes or to determine the amount of particular types of exosomes present within a mammal (e.g., a human). The presence or amount of particular types of distinct expression products, such as miRNAs within an exosomes comprising sample can indicate that the mammal has a particular disease or disorder. As indicated hereinbefore, for successful pregnancy, modulation of the maternal immune system is necessary. Experimental results described in detail further below indicate that specific miRNAs, such as miRNAs of the C19MC as well as of the miR-371-3 cluster are such important modulators of the maternal immune system. The presence or amount of particular types of these miRNAs within an exosomes comprising sample may be used thus for the estimation of ability of the embryo to implant or of a sperm sample to fertilize if the exosomes were isolated from a supernatant of cell culture medium of blastocysts or embryos. Therefore, the present invention also relates to a method for obtaining exosomes for use in immunomodulation comprising isolation and purification of exosomes from a supernatant of cell cultures of embryonic or fetal cells expressing miRNAs of the C19MC cluster, the miR-371-373 cluster and/or the miR302-367 cluster as defined hereinabove and below and as enlisted in Tables 1 and 2 above.

By the methods of the present invention, exosomes can be isolated from cell cultures of different cell types including cell types associated with embryonal implantation and development. Methods of isolation, purification and culturing of umbilical cord, amniotic, placental and chorionic cells from a biological sample are known in the art and described, e.g., in international applications WO2005/001081, WO 2000/073421, WO2002/046373 and WO2003/042405, the disclosure content of which is incorporated herein by reference and can be used to obtain cell cultures of interest for the isolation of exosomes. In one embodiment of the present invention the aforementioned method for obtaining exosomes is provided, wherein the cell cultures are selected from the group of cells comprising cells from the umbilical cord, the amniotic membrane, the placenta and chorionic membrane.

Furthermore, the present invention relates to a method for obtaining exosomes for use in immunomodulation from a biological sample comprising the steps of setting up a cell culture from the biological sample, collecting the supernatant of the cell culture and isolating and purifying the exosomes thereof.

The biological samples for obtaining exosomes may be isolated from cells, cell culture, tissue, animal or human patient with the same genetical background as those envisaged for the immunomodulating treatment with the obtained exosomes or their content, or from cells, cell culture, tissue, organism, animal or human patient with a different genetical background as those envisaged for the treatment. In this respect, in one embodiment the method for obtaining exosomes for use in immunomodulation from a biological sample is provided, wherein the biological sample comprises autologous cells, tissue sample or aspirate. In another embodiment the method for obtaining exosomes for use in immunomodulation from a biological sample is provided, wherein the biological sample comprises allologous cells, tissue sample or aspirate.

The present invention further relates to a method for in vitro generation of exosomes comprising miRNAs, binding molecules and/or double stranded nucleic acids as defined hereinabove and below and exosomes obtained according to the aforementioned methods for use in immunomodulation. Methods for producing artificial exosomes are known in the art as well.

Such artificial exosomes can be derived from coated liposomes as described in De La Peña et al., J. Immunol. Methods. 344 (2009), 121-132.

Exosomes obtained according to the methods of the present invention can be used in immunomodulation in patients suffering from several diseases and disease types. In one embodiment of the present invention exosomes obtained according to the aforementioned methods are provided for use in treatment of an autoimmune disease.

In regard of the treatment of a particular disease, the exosomes of the present invention can be administered on distinct routes of administration depending on the intention whether the effect is local (in "topical" administration) or systemic (in "enteral" or "parenteral" administration) and can be formulated in accordance to the specific administration route requirements. Therefore, in one embodiment of the present invention, exosomes obtained according to the aforementioned methods are provided prepared for use in treatment of an autoimmune disease by a local administration. In another embodiment of the present invention the exosomes prepared for use in treatment of an autoimmune disease are administered using joint injection, preferably intra-articular injection or intra-nasal application. In a further embodiment of the present invention the exosomes obtained according to the aforementioned methods are prepared for use in treatment of an autoimmune disease by a systemic administration.

Prior to isolation of exosomes, the cells, tissue, organs or animals the exosomes are isolated from can be genetically engineered to express, or to overexpress specific miRNAs such as the aforementioned miRNAs and/or may be exposed to one or more agents, such as Azacytidine (5-Aza-2'-deoxycytidine) or other DNA demethylating agents, which are also capable of enhancing the ability of the cells to secrete exosomes (see, e.g., Xiao et al., (2010) "Effect of 5-aza-2'-deoxycytidine on immune-associated proteins in exosomes from hepatoma.". World J Gastroenterol. 16, 2371-2377.). The exposition to such agents may be performed as described for Azacytidine on page 2372 in the "Cell culture" subsection of the "Materials and Methods" section of Xiao et al., (2010) the disclosure content of which is hereby incorporated by reference.

Therefore, the present invention further relates to the use of Azacytidine or other DNA demethylating agents for the treatment of cell cultures in the aforementioned methods for obtaining or generation of exosomes to enhance the ability of the cells to secrete exosomes.

Furthermore, the present invention also relates to Azacytidine or other DNA demethylating agents for the use in treatment of tissues in vivo to enhance their ability to secrete exosomes enhanced for miRNAs of the C19MC, the miR-371-373 and/or the miR302-367 cluster as defined hereinabove and below and enlisted in Tables 1 and 2.

As indicated above, the presence or amount of particular types of these miRNAs within an exosomes comprising sample can be used for the estimation of the ability of the embryo to implant. In this respect, e.g., a supernatant of the respective cell culture medium can be isolated and analyzed for the presence and amount of miRNAs of the miR-371-3 and C19MC clusters as defined in Table 1 above. The results of this analysis can be then used to estimate the implantation probability of an embryo.

Therefore, the present invention further relates to a method to diagnose the ability of the embryo to implant or of a sperm sample to fertilize comprising identification of at least one miRNA of the C19MC cluster and/or the miR-371-373 cluster as defined hereinabove and below and enlisted in Table 1 in cell culture medium of blastocysts or embryos or in seminal fluid, respectively.

In one embodiment of the present invention the method to diagnose as defined above is provided, wherein a comparable or increased level of the at least one miRNA of the C19MC cluster and/or the miR-371-373 cluster as defined herein and enlisted in Table 1 compared to a control sample is indicative of a normal or increased ability of the embryo to implant or of a sperm sample to fertilize and a decreased level of the at least one miRNA compared to a control sample is indicative of a reduced ability of the embryo to implant or of a sperm sample to fertilize.

The method to diagnose as defined above, wherein the presence of the at least one miRNA of the C19MC cluster and/or the miR-371-373 cluster as defined herein and enlisted in Table 1 is indicative of a normal or increased ability of the embryo to implant or of a sperm sample to fertilize and the absence of the at least one miRNA of said cluster is indicative of a reduced ability of the embryo to implant or of a sperm sample to fertilize.

### Definitions and embodiments:

It is to be noted that the term "a" or "an" entity refers to one or more of that entity; for example, "an polypeptide," is understood to represent one or more polypeptides. As such, the terms "a" (or "an"), "one or more," and "at least one" can be used interchangeably herein.

Unless stated otherwise, the terms "disorder" and "disease" are used interchangeably herein. By "subject" or "individual" or "animal" or "patient" or "mammal", is meant any subject, particularly a mammalian subject, e.g., a human patient, for whom diagnosis, prognosis, prevention, or therapy is desired.

The terms "microRNA" (miRNA) as used in the present invention relates to a non-protein coding RNAs, which may comprise 13-35, 18-25 or 21-24 nucleotides, generally of between about 19 to about 25 nucleotides, preferably of about 22 nucleotides as the majority of the naturally occurring miRNAs, that guide cleavage in trans of target transcripts, negatively regulating the expression of genes involved in various regulation and development pathways (Bartel DP., Cell 116 (2004), 281-297; He and Hannon, Nat. Rev. Genet. 5 (2004), 522-531; Lagos-Quintana et al., Science 294 (2001), 853-858; Ambros V., Cell, 113 (2003), 673-676). Bases 2-8 of the mature miRNA are defined herein as the "miRNA seed sequence". The complete complementarity of 6 to 8 bp between the target RNA sequence and the miRNA seed sequence is a major determinant of miRNA target recognition. Younger and Corey, Nucleic Acids Res. 39 (2011), 5682-5691. The sequence requirements for mature miRNA binding to a recognition site, and methods for predicting miRNA binding to a given sequence, are discussed, for example, in Lewis et al., Cell 115 (2003), 787-798; John et al., PLoS Biol 2(11): e363 (2004)

Some miRNA genes (MIR genes) have been identified and made publicly available in a database ('miRBase", available on line at microrna.sanger.ac.uk/ sequences). Additional MIR genes and mature miRNAs are also described in U.S. Patent Application Publication 2005/0120415. MIR genes have been reported to occur in inter-genic regions, both isolated and in clusters in the genome, but can also be located entirely or partially within introns of other genes (both protein-coding and non-protein-coding; Saini et al., Proc Natl Acad Sci U S A 104 (2007), 17719-17724). For a recent review of miRNA biogenesis, see Kim VN., Nature Rev. Mol. Cell Biol . 6 (2005), 376-385. Transcription of MIR genes can be, at least in some cases, under promotional control of a MIR gene's own promoter. Transcription is probably generally mediated by RNA polymerase II then (Lee et al., Embo J 23 (2004), 4051-4060). The primary transcript (which can be polycistronic) termed a "pri-miRNA", a miRNA precursor molecule that can be quite large (several kilobases) and contains one or more local double-stranded or "hairpin" regions as well as the usual 5' "cap" and polyadenylated tail of an mRNA. See, for example, FIG. 1 in Kim, Nature Rev. Mol. Cell Biol. 6 (2005), 376-385.

This pri-miRNA is believed to be "cropped" by the nuclear RNase III Drosha to produce a shorter (~70 nucleotides) miRNA precursor molecule known as a "pre-miRNA". Following nuclear processing by Drosha, pre-miRNAs are exported to the nucleus where the enzyme Dicer generates the short, mature miRNAs. See, for example, Lee et al. EMBO Journal 21 (2002), 4663-4670; Reinhart et al., Genes & Dev., 16 (2002):1616-1626; Lund et al., Science 303 (2004), 95-98; and Millar and Waterhouse, Funct. Integr Genomics 5 (2005), 129-135. MicroRNAs can thus be described in terms of RNA (e.g., RNA sequence of a mature miRNA or a miRNA precursor RNA molecule), or in terms of DNA (e.g., DNA sequence corresponding to a mature miRNA, RNA sequence or DNA sequence encoding a MIR gene or fragment of a MIR gene or a miRNA precursor).

MIR gene families appear to be substantial, estimated to account for 1% of at least some genomes and capable of influencing or regulating expression of about a third of all genes (see, for example, Tomari et al., Curr. Biol., 15(2005), R61-64; Tang G., Trends Biochem. Sci., 30 (2005), 106-14; Kim Nature Rev. Mol. Cell Biol., 6 (2005), 376-385). miRNAs are involved, for example, in regulation of cellular differentiation, proliferation and apoptosis, and are probably involved in the pathology of at least some diseases, including cancer, where miRNAs may function variously as oncogenes or as tumor suppressors. See, for example, O'Donnell et al., Nature 435 (2005), 839-843; Cai et al., Proc. Natl. Acad. Sci. USA , 102 (2005), 5570-5575; Morris and McManus (2005) Sci. STKE , pe41 (available online at stke.sciencemag.org/cgi/reprint/sigtrans;2005/297/pe41.pdf). MicroRNA (MIR) genes have identifying characteristics, including conservation among plant species, a stable foldback structure, and processing of a specific miRNA/ miRNA* duplex by Dicer-like enzymes (Ambros et al. (2003) RNA, 9:277-279). These characteristics have been used to identify miRNAs and their corresponding genes by supplementing molecular cloning by systematic computational approaches that identify evolutionarily conserved miRNA genes by searching for patterns of sequence and secondary structure conservation that are characteristic of metazoan miRNA hairpin precursors (Ambros et al., Curr. Biol., 13 (2003), 807-818; Grad et al., Mol. Cell 11 (2003), 1253-1263; Lai et al., Curr. Biol. 13 (2003), R925-R936; Lim et al., Science 299 (2003), 1540 and Lim et al., Genes Dev., 17 (2003), 991-1008. Publicly available microRNA genes are catalogued at miRBase (Griffiths-Jones et al. (2003) Nucleic Acids Res., 31:439-441). According to the present invention the term "miRNA precursor" refers interchangeably to all precursor forms of a mature miRNA, i.e. pri-miRNA and pre-miRNA.

Recognition sites of miRNAs have been validated in all regions of a mRNA, including the 5' untranslated region, coding region, and the 3' untranslated region, indicating that the position of the miRNA target site relative to the coding sequence may not necessarily affect suppression (see, for example, Rhoades et al. (2002) Cell, 110:513-520; Yekta et al., Science 304 (2004), pp. 594-596; Davis et al. Curr. Biol., 15 (2005), 743-749; Lewis et al., Cell 115 (2003), 787-798; Lewis et al., Cell 120 (2005), 15-20; Baek et al., Nature 455 (2008), 64-71 and Selbach et al., Nature 455 (2008), 58-63.

Binding between the mRNA and the miRNA has not to be perfect, some mismatches have been observed without any effect on the miRNAs efficiency. In this respect, experimentally verified miRNA target sites indicate that the 5' end of the miRNA tends to have more bases complementary to the target than its 3' end (Moss et al., Cell 88 (1997), 637-646; Johnston and Hobert, Nature 426 (2003), 845-849; John et al., PLoS Biol 2(11): e363 (2004)). Therefore, as a micro-RNA of the present invention, a micro-RNA consisting of a nucleotide sequence having an identity of 80% or more to the nucleotide sequence of any one of SEQ ID. NO 1 to 66 and 68, 69, 71, 72, 74, 75, 77 and 78, preferably a micro-RNA consisting of a nucleotide sequence having an identity of 90% or more, more preferably 95% or more, can be mentioned and referred to as a variant thereof.

As a precursor micro-RNA of the present invention, a precursor micro-RNA consisting of a nucleotide sequence having an identity of 80% or more to the nucleotide sequence of any one precursor miRNA of SEQ ID. NOs 1 to 66 as indicated in the miRBase within the entries of the respective miRNA molecules of the present invention indicated in Table 1 and of SEQ ID Nos.: 67, 70, 73 or 76, preferably a micro-RNA consisting of a nucleotide sequence having an identity of 90% or more, more preferably 95% or more, can be mentioned and referred to as a variant thereof.

The variant may also be a nucleotide sequence which hybridizes under stringent conditions to the referenced nucleotide sequence, complements thereof, or nucleotide sequences substantially identical thereto. As will be appreciated by those in the art, the depiction of a single strand also defines the sequence of the complementary strand. Thus, a nucleic acid also encompasses the complementary strand of a depicted single strand. As will also be appreciated by those in the art, many variants of a nucleic acid may be used for the same purpose as a given nucleic acid. Thus, a nucleic acid also encompasses substantially identical nucleic acids and complements thereof. As will also be appreciated by those in the art, a single strand provides a probe for a probe that may hybridize to the target sequence under stringent hybridization conditions. Thus, a nucleic acid also encompasses a probe that hybridizes under stringent hybridization conditions.

"Probe" as used herein may mean an oligonucleotide capable of binding to a target nucleic acid of complementary sequence through one or more types of chemical bonds, usually through complementary base pairing, usually through hydrogen bond formation. Probes may bind target sequences lacking complete complementarity with the probe sequence depending upon the stringency of the hybridization conditions. There may be any number of base pair mismatches which will interfere with hybridization between the target sequence and the single stranded nucleic acids of the present invention. However, if the number of mutations is so great that no hybridization can occur under even the least stringent of hybridization conditions, the sequence is not a complementary target sequence.

"Stringent hybridization conditions" used herein may mean conditions under which a first nucleic acid sequence (e.g., probe) will hybridize to a second nucleic acid sequence (e.g., target), such as in a complex mixture of nucleic acids, but to no other sequences. Stringent conditions are sequence-dependent and will be different in different circumstances. Generally, stringent conditions are selected to be about 5-10° C. lower than the thermal melting point (Tm) for the specific sequence at a defined ionic strength pH. The Tm may be the temperature (under defined ionic strength, pH, and nucleic concentration) at which 50% of the probes complementary to the target hybridize to the target sequence at equilibrium (as the target sequences are present in excess, at Tm, 50% of the probes are occupied at equilibrium). Stringent conditions may be those in which the salt concentration is less than about 1.0 M sodium ion, typically about 0.01-1.0 M sodium ion concentration (or other salts) at pH 7.0 to 8.3 and the temperature is at least about 30° C for short probes (e.g., about 10-50 nucleotides) and at least about 60° C for long probes (e.g., greater than about 50 nucleotides). Stringent conditions may also be achieved with the addition of destabilizing agents such as formamide. For selective or specific hybridization, a positive signal may be at least 2 to 10 times background hybridization. Exemplary stringent hybridization conditions include the following: 50% formamide, 5×SSC, and 1% SDS, incubating at 42° C., or, 5×SSC, 1% SDS, incubating at 65° C., with wash in 0.2×SSC, and 0.1% SDS at 65° C.

"Substantially complementary" used herein may mean that a first sequence is at least 60%, 65%, 70%, 75%, 80%, 85%, 90%, 95%, 97%, 98% or 99% identical to the complement of a second sequence over a region of 8, 9, 10, 11, 12, 13, 14, 15, 16, 17, 18, 19, 20, 21, 22, 23, 24, 25, 30, 35, 40, 45, 50 or more nucleotides, or that the two sequences hybridize under stringent hybridization conditions.

"Substantially identical" used herein may mean that a first and second sequence are at least 60%, 65%, 70%, 75%, 80%, 85%, 90%, 95%, 97%, 98% or 99% identical over a region of 8, 9, 10, 11, 12, 13, 14, 15, 16, 17, 18, 19, 20, 21, 22, 23, 24, 25, 30, 35, 40, 45, 50 or more nucleotides or amino acids, or with respect to nucleic acids, if the first sequence is substantially complementary to the complement of the second sequence.

As used herein, the term "binding molecule" refers to any agent (e.g., peptide, protein, nucleic acid polymer, aptamer, spiegelmer or small molecule) that specifically binds to a target of interest. In a particular preferred embodiment the term "binding molecule" in the sense of the present invention includes synthetic miRNA mimics, RNA-molecules, antibodies, aptamers, spiegelmers, and modified variants of siRNA, miRNA or a variant thereof. Such a variant may be chemically synthesised and may have advantages for RNA silencing-related processes. Some of these modifications may help protect the miRNA-related molecule from degradation, such as a 2'-o-methyl, 2'-o-allyl, 2'-deoxy-fluorouridine modification, or phosphorothioates. Other modifications may also help increase the affinity of the miRNA-related molecule for its target or reduce its off-target effects, such as the locked-nucleic acid modification, in which a methylene bridge connects the 2'-oxygen with the 4'-carbon of the ribose ring. Other modifications may enhance the loading of the correct strand of a siRNA or miRNA into the Argonaute complex (Hammell CM., RNA Biol. 5 (2008), 123-127), such as by adding a 5' phosphate or methyl to one strand of a doublestranded miRNA/miRNA*complex (miRNA* is the miRNA's partner strand that arises from the opposite arm in the precursor).

The target of interest is herein defined as the recognition site of the miRNA molecules of the present invention as defined hereinabove, preferably within a nucleic acid, most preferably a pre-mRNA or mRNA-molecule. The overall effect of such interferences with a target nucleic acids' function is a specific modulation of the expression of said gene. In the context of the present invention, "modulation" means either an increase (stimulation) or a decrease (inhibition) in the expression of a gene. In the context of the present invention, in particular concerning "immunomodulation", inhibition is the preferred form of modulation of gene expression. Because an mRNA having a nucleotide sequence complementary to the nucleotides 2 to 8 on the 5' terminal side of a microRNA (the seed sequence) undergoes suppression of the translation thereof by the micro-RNA (Current Biology, 15, R458-R460 (2005)), a nucleotide sequence complementary to the seed sequence of a micro-RNA of the present invention can be mentioned as a target nucleotide sequence of the micro-RNA or binding molecule of the present invention.

"miRNA mimics" are nonnatural double-stranded miRNA-like RNA fragments. They are designed to have the 5'-end bearing a partially complementary motif to the selected sequence in the 3'UTR unique to the target gene. Introduced into cells, a miRNA mimic can bind specifically to its target gene and produce posttranscriptional repression, more specifically translational inhibition, of the gene. Unlike endogenous miRNAs which may target several genes at once, miR-Mimics act in a gene-specific fashion (Wang, Methods Mol Biol. 676 (2011), 211-223; Xiao et al., J Cell Physiol 212 (2007), 285-292).

As used herein, the term "aptamer" refers to a DNA or RNA molecule that has been selected from random pools based on their ability to bind other molecules with high affinity specificity based on non- Watson and Crick interactions with the target molecule (see, e.g., Cox and Ellington, Bioorg. Med. Chem. 9 (2001), 2525-2531; Lee et al., Nuc. Acids Res. 32 (2004), D95-D100). Aptamers can be selected which bind nucleic acid, proteins, small organic compounds, vitamins, inorganic compounds, cells, and even entire organisms.

The peptides and aptamers of the present invention are synthesized by any suitable method. For example, targeting peptides and aptamers of the present invention can be chemically synthesized by solid phase peptide synthesis. Techniques for solid phase synthesis are described, for example, by Barany and Merrifield (1979) Solid-Phase Peptide Synthesis; pp. 1-284 in The Peptides: Analysis, Synthesis, Biology, (Gross, and Meinehofer, eds.), Academic, New York, Vol. 2, Special Methods in Peptide Synthesis, Part A.; Merrifield, J. Am. Chem. Soc, 85 (1963), 2149-2154; and Stewart and Young (1984) Solid Phase Peptide Synthesis, 2nd ed. Pierce Chem. Co., Rockford, Illinois.

Spiegelmers are nucleic acids comprising a number of L-nucleotides which show binding activities towards a target or a part thereof. The basic method of spiegelmer generation is subject to the international patent application WO 1998/008856 the disclosure of which is incorporated herein by reference. Basically, this method relies on the so-called SELEX technique as described, e. g., in US 5,475,096. The method uses combinatorial DNA or RNA libraries comprising a randomised stretch of about 10 to about 100 nucleotides which are flanked by two primer binding regions at the 5'and 3'end. The generation of such combinatorial libraries is, for example, described in Conrad et al., Methods Enzymol., 267 (1996),336-367. Such a chemically synthesized single-stranded DNA library may be transferred into a double-stranded library via polymerase chain reaction.

Such a library may already be used for selection purpose. The selection occurs such that the, typically single-stranded, library is contacted with a target molecule and the binding elements of the library are then amplified. By repeating these steps several times oligonucleotide molecules may be generated having a significant binding activity towards the target used.

"Antibodies" are generated by state of the art procedures, e.g., as described in Tijssen (Tijssen, P., Practice and theory of enzyme immunoassays, 11, Elsevier Science Publishers B.V., Amsterdam, the whole book, especially pages 43-78). The antibody of the present invention may exist in a variety of forms besides complete antibodies; including, for example, an F(ab') fragment, an F(ab) fragment, and an F(ab')₂ fragment, or any other antigen-binding fragment, as well as in single chains; see e.g. international applications WO88/09344, WO 2005/003169, WO 2005/003170 and WO 2005/003171.

Antibodies which may be used according to the present invention include immunoglobulin molecules and immunologically active portions of immunoglobulin molecules, *i.e.* molecules that contain an antigen binding site that specifically binds an antigen. The immunoglobulin molecules of the invention can be of any class (*e.g.* IgG, IgE, IgM, IgD or IgA) or subclass of immunoglobulin molecule.

### Polynucleotides:

The term "polynucleotide" is intended to encompass a singular nucleic acid as well as plural nucleic acids, and refers to an isolated nucleic acid molecule or construct, e.g., messenger RNA (mRNA) or plasmid DNA (pDNA). A polynucleotide may comprise a conventional phosphodiester bond or a non-conventional bond (*e.g.,* an amide bond, such as found in peptide nucleic acids (PNA)). The term "nucleic acid" or "double-stranded nucleic acid" refers to any one or more nucleic acid segments, e.g., DNA or RNA fragments, present in a polynucleotide, comprising preferably the sequence encoding at least one miRNA molecule of the present invention. By "isolated" nucleic acid or polynucleotide is intended a nucleic acid molecule, DNA or RNA, which has been removed from its native environment. For example, a recombinant polynucleotide encoding at least one miRNA or an antibody contained in a vector is considered isolated for the purposes of the present invention. Further examples of an isolated polynucleotide include recombinant polynucleotides maintained in heterologous host cells or purified (partially or substantially) polynucleotides in solution. Isolated RNA molecules include *in vivo* or *in vitro* RNA transcripts of polynucleotides of the present invention. Isolated polynucleotides or nucleic acids according to the present invention further include such molecules produced synthetically. In addition, a polynucleotide or a nucleic acid may be or may include a regulatory element such as a promoter, ribosome binding site, or a transcription terminator.

As used herein, a "coding region" is a portion of nucleic acid which consists of codons translated into amino acids or comprising the sequence of a functional RNA, such as tRNA, rRNA, catalytic RNA, precursor of a miRNA molecule, miRNA, siRNA and antisense RNA. Although a "stop codon" (TAG, TGA, or TAA) is not translated into an amino acid, it may be considered to be part of a coding region, but any flanking sequences, for example promoters, ribosome binding sites, transcriptional terminators, introns, and the like, are not part of a coding region. This distinction does of course not refer to sequences encoding miRNAs which have been extracted from their original loci in such kind of sequences, e.g., intronic sequences of protein encoding genes, but to the function of the sequences in the polynucleotide constructs of the present invention. A coding region may also be an mRNA or cDNA corresponding to the coding regions (e.g., exons and miRNA) optionally comprising 5'- or 3'-untranslated sequences linked thereto. A coding region may also be an amplified nucleic acid molecule produced in vitro comprising all or a part of the coding region and/or 5'- or 3'-untranslated sequences linked thereto.

Two or more coding regions of the present invention can be present in a single polynucleotide construct, e.g., on a single vector, or in separate polynucleotide constructs, e.g., on separate (different) vectors. Furthermore, any vector may contain a single coding region, or may comprise two or more coding regions, e.g., a single vector may separately encode an immunoglobulin heavy chain variable region and an immunoglobulin light chain variable region. In addition, a vector, polynucleotide, or nucleic acid of the invention may encode heterologous coding regions, either fused or not fused to a nucleic acid encoding a binding molecule, an antibody, or fragment, variant, or derivative thereof. Heterologous coding regions include without limitation specialized elements or motifs, such as a secretory signal peptide or a heterologous functional domain.

In certain embodiments, the polynucleotide or nucleic acid is DNA. In the case of DNA, a polynucleotide comprising a nucleic acid which encodes a miRNA or polypeptide normally may include a promoter and/or other transcription or translation control elements operably associated with one or more coding regions. An operable association exists when a coding region for a gene product, *e.g*., a polypeptide, is associated with one or more regulatory sequences in such a way as to place expression of the gene product under the influence or control of the regulatory sequence(s). Two DNA fragments (such as a miRNA/polypeptide coding region and a promoter associated therewith) are "operably associated" or "operably linked" if induction of promoter function results in the transcription of mRNA encoding the desired gene product and if the nature of the linkage between the two DNA fragments does not interfere with the ability of the expression regulatory sequences to direct the expression of the gene product or interfere with the ability of the DNA template to be transcribed. Thus, a promoter region would be operably associated with a nucleic acid encoding a miRNA/polypeptide if the promoter was capable of effecting transcription of that nucleic acid. The promoter may be a cell-specific promoter that directs substantial transcription of the DNA only in predetermined cells. Other transcription control elements, besides a promoter, for example enhancers, operators, repressors, and transcription termination signals, can be operably associated with the polynucleotide to direct cell-specific transcription. Suitable promoters and other transcription control regions are disclosed herein.

A variety of transcription control regions are known to those skilled in the art. These include, without limitation, transcription control regions which function in vertebrate cells, such as, but not limited to, promoter and enhancer segments from cytomegaloviruses (the immediate early promoter, in conjunction with intron-A), simian virus 40 (the early promoter), and retroviruses (such as Rous sarcoma virus). Other transcription control regions include those derived from vertebrate genes such as actin, heat shock protein, bovine growth hormone and rabbit β-globin, as well as other sequences capable of controlling gene expression in eukaryotic cells. Additional suitable transcription control regions include tissue-specific promoters and enhancers as well as lymphokine-inducible promoters (*e.g.*, promoters inducible by interferons or interleukins).

Similarly, a variety of translation control elements are known to those of ordinary skill in the art. These include, but are not limited to ribosome binding sites, translation initiation and termination codons, and elements derived from picornaviruses (particularly an internal ribosome entry site, or IRES, also referred to as a CITE sequence).

In a particularly preferred embodiment, a polynucleotide of the present invention is RNA, for example, in the form of messenger RNA (mRNA), micro RNA (miRNA), miRNA precursor, small hairpin RNA (shRNA), small interfering RNA (siRNA) or any other RNA product.

### Determination of similiarity and/or identity of molecules:

"Similarity" between two peptides is determined by comparing the amino acid sequence of one peptide to the sequence of a second peptide. An amino acid of one peptide is similar to the corresponding amino acid of a second peptide if it is identical or a conservative amino acid substitution. Conservative substitutions include those described in Dayhoff, M.O., ed., The Atlas of Protein Sequence and Structure 5, National Biomedical Research Foundation, Washington, D.C. (1978), and in Argos, EMBO J. 8 (1989), 779-785. For example, amino acids belonging to one of the following groups represent conservative changes or substitutions: -Ala, Pro, Gly, Gln, Asn, Ser, Thr; -Cys, Ser, Tyr, Thr; -Val, Ile, Leu, Met, Ala, Phe; -Lys, Arg, His; -Phe, Tyr, Trp, His; and -Asp, Glu.

The determination of percent identity or similarity between two sequences is preferably accomplished using the mathematical algorithm of Karlin and Altschul (1993) Proc. Natl. Acad. Sci USA 90: 5873-5877. Such an algorithm is incorporated into the BLASTn and BLASTp programs of Altschul et al. (1990) J. Mol. Biol. 215: 403-410 available at NCBI (http://www.ncbi.nlm.nih.gov/blast/Blast.cge).

The determination of percent identity or similarity is performed with the standard parameters of the BLASTn and BLASTp programs, as recommended on the NCBI webpage and in the "BLAST Program Selection Guide" in respect of sequences of a specific length and composition.

BLAST polynucleotide searches are performed with the BLASTn program.

For the general parameters, the "Max Target Sequences" box may be set to 100, the "Short queries" box may be ticked, the "Expect threshold" box may be set to 1000 and the "Word Size" box may be set to 7 as recommended for short sequences (less than 20 bases) on the NCBI webpage. For longer sequences the "Expect threshold" box may be set to 10 and the "Word Size" box may be set to 11. For the scoring parameters the "Match/mismatch Scores" may be set to 1,-2 and the "Gap Costs" box may be set to linear. For the Filters and Masking parameters, the "Low complexity regions" box may not be ticked, the "Species-specific repeats" box may not be ticked, the "Mask for lookup table only" box may be ticked, the "DUST Filter Settings" may be ticked and the "Mask lower case letters" box may not be ticked. In general the "Search for short nearly exact matches" may be used in this respect, which provides most of the above indicated settings. Further information in this respect may be found in the "BLAST Program Selection Guide" published on the NCBI webpage.

BLAST protein searches are performed with the BLASTp program. For the general parameters, the "Max Target Sequences" box may be set to 100, the "Short queries" box may be ticked, the "Expect threshold" box may be set to 10 and the "Word Size" box may be set to "3". For the scoring parameters the "Matrix" box may be set to "BLOSUM62", the "Gap Costs" Box may be set to "Existence: 11 Extension: 1", the "Compositional adjustments" box may be set to "Conditional compositional score matrix adjustment". For the Filters and Masking parameters the "Low complexity regions" box may not be ticked, the "Mask for lookup table only" box may not be ticked and the "Mask lower case letters" box may not be ticked.

Modifications of both programs, e.g., in respect of the length of the searched sequences, are performed according to the recommendations in the "BLAST Program Selection Guide" published in a HTML and a PDF version on the NCBI webpage.

### Diseases and disorders:

Unless stated otherwise, the terms "disorder" and "disease" are used interchangeably herein. The term "autoimmune disorder" as used herein is a disease or disorder arising from and directed against an individual's own tissues or organs or a co-segregate or manifestation thereof or resulting condition therefrom. Autoimmune diseases are primarily caused by dysregulation of adaptive immune responses and autoantibodies or autoreactive T cells against self-structures are formed. Nearly all autoimmune diseases have an inflammatory component, too. Autoinflammatory diseases are primarily inflammatory, and some classic autoinflammatory diseases are caused by genetic defects in innate inflammatory pathways. In autoinflammatory diseases, no autoreactive T cells or autoantibodies are found. In many of these autoimmune and autoinflammatory disorders, a number of clinical and laboratory markers may exist, including, but not limited to, hypergammaglobulinemia, high levels of autoantibodies, antigen-antibody complex deposits in tissues, benefit from corticosteroid or immunosuppressive treatments, and lymphoid cell aggregates in affected tissues. Without being limited to a theory regarding B-cell mediated autoimmune disorder, it is believed that B cells demonstrate a pathogenic effect in human autoimmune diseases through a multitude of mechanistic pathways, including autoantibody production, immune complex formation, dendritic and T-cell activation, cytokine synthesis, direct chemokine release, and providing a nidus for ectopic neo-lymphogenesis. Each of these pathways may participate to different degrees in the pathology of autoimmune diseases.

As used herein, an "autoimmune disorder" can be an organ-specific disease (i.e., the immune response is specifically directed against an organ system such as the endocrine system, the hematopoietic system, the skin, the cardiopulmonary system, the gastrointestinal and liver systems, the renal system, the thyroid, the ears, the neuromuscular system, the central nervous system, etc.) or a systemic disease that can affect multiple organ systems (for example, systemic lupus erythematosus (SLE), rheumatoid arthritis, polymyositis, autoimmune polyendocrinopathy syndrome etc. Preferred such diseases include Acute disseminated encephalomyelitis (ADEM), Alopecia areata, Ankylosing Spondylitis, Antiphospholipid syndrome (APS), Autoimmune cardiomyopathy, Autoimmune hemolytic anemia, Autoimmune hepatitis, Autoimmune inner ear disease, Autoimmune lymphoproliferative syndrome (ALPS), Autoimmune peripheral neuropathy, Autoimmune pancreatitis, Autoimmune polyendocrine syndrome, Autoimmune progesterone dermatitis, Autoimmune thrombocytopenic purpura, Autoimmune urticaria, Autoimmune uveitis, Celiac disease, Cold agglutinin disease, Crohns Disease, Dermatomyositis, Diabetes mellitus type 1, Endometriosis, Eosinophilic fasciitis, Gastrointestinal pemphigoid, Goodpasture's syndrome, Graves' disease, Guillain-Barré syndrome (GBS), Hashimoto's encephalopathy, Hashimoto's thyroiditis, Idiopathic thrombocytopenic purpura (Autoimmune thrombocytopenic purpura), Lupus erythematosus, Miller-Fisher syndrome (Guillain-Barre-Syndrome), Mixed Connective Tissue Disease, Myasthenia gravis, Pemphigus vulgaris, Pernicious anaemia, Polymyositis, Primary biliary cirrhosis, Psoriasis, Psoriatic arthritis, Relapsing polychondritis, Rheumatoid arthritis, Sjögren's syndrome, Temporal arteritis ("giant cell arteritis"), Transverse myelitis, Ulcerative colitis, Undifferentiated connective tissue disease (Mixed connective tissue disease), Vasculitis, Wegener's granulomatosis.

### Treatment and drugs:

As used herein, the terms "treat" or "treatment" refer to both therapeutic treatment and prophylactic or preventative measures, wherein the object is to prevent or slow down (lessen) an undesired physiological change or disorder, such as the development of an autoimmune and/or autoinflammatory disease. Beneficial or desired clinical results include, but are not limited to, alleviation of symptoms, diminishment of extent of disease, stabilized (i.e., not worsening) state of disease, delay or slowing of disease progression, amelioration or palliation of the disease state, and remission (whether partial or total), whether detectable or undetectable. "Treatment" can also mean prolonging survival as compared to expected survival if not receiving treatment. Those in need of treatment include those already with the condition or disorder as well as those prone to have the condition or disorder or those in which the manifestation of the condition or disorder is to be prevented.

If not stated otherwise the term "drug," "medicine," or "medicament" are used interchangeably herein and shall include but are not limited to all (A) articles, medicines and preparations for internal or external use, and any substance or mixture of substances intended to be used for diagnosis, cure, mitigation, treatment, or prevention of disease of either man or other animals; and (B) articles, medicines and preparations (other than food) intended to affect the structure or any function of the body of man or other animals; and (C) articles intended for use as a component of any article specified in clause (A) and (B). The term "drug," "medicine," or "medicament" shall include the complete formula of the preparation intended for use in either man or other animals containing one or more "agents," "compounds", "substances" or "(chemical) compositions" as and in some other context also other pharmaceutically inactive excipients as fillers, disintegrants, lubricants, glidants, binders or ensuring easy transport, disintegration, disaggregation, dissolution and biological availability of the "drug," "medicine," or "medicament" at an intended target location within the body of man or other animals, e.g., at the skin, in the stomach or the intestine. The terms "agent," "compound", or "substance" are used interchangeably herein and shall include, in a more particular context, but are not limited to all pharmacologically active agents, *i.e.* agents that induce a desired biological or pharmacological effect or are investigated or tested for the capability of inducing such a possible pharmacological effect by the methods of the present invention.

The term "immunomodulation" as used according to the present invention refers to an alteration of the immune response by augmenting (immunopotentiation) or reducing (immunosuppression) the ability of the immune system to produce antibodies or sensitized cells that recognize and react with the antigen that initiated their production. Several substances suitable for immunomodulation are known in the art, e.g., corticosteroids, cytotoxic agents, thymosin, and immunoglobulins.

### Pharmaceutical carriers:

Pharmaceutically acceptable carriers and administration routes can be taken from corresponding literature known to the person skilled in the art. The pharmaceutical compositions of the present invention can be formulated according to methods well known in the art; see for example Remington: The Science and Practice of Pharmacy (2000) by the University of Sciences in Philadelphia, ISBN 0-683-306472, Vaccine Protocols.2nd Edition by Robinson et al., Humana Press, Totowa, New Jersey, USA, 2003; Banga, Therapeutic Peptides and Proteins: Formulation, Processing, and Delivery Systems. 2nd Edition by Taylor and Francis. (2006), ISBN: 0-8493-1630-8. Examples of suitable pharmaceutical carriers are well known in the art and include phosphate buffered saline solutions, water, emulsions, such as oil/water emulsions, various types of wetting agents, sterile solutions etc. Compositions comprising such carriers can be formulated by well-known conventional methods. These pharmaceutical compositions can be administered to the subject at a suitable dose. Administration of the suitable compositions may be effected by different ways. Examples include administering a composition containing a pharmaceutically acceptable carrier via oral, intranasal, rectal, topical, intraperitoneal, intravenous, intramuscular, subcutaneous, subdermal, transdermal, intrathecal, and intracranial methods. Aerosol formulations such as nasal spray formulations include purified aqueous or other solutions of the active agent with preservative agents and isotonic agents. Such formulations are preferably adjusted to a pH and isotonic state compatible with the nasal mucous membranes. Pharmaceutical compositions for oral administration, such as single domain antibody molecules (e.g., "nanobodies^{™}") etc are also envisaged in the present invention. Such oral formulations may be in tablet, capsule, powder, liquid or semi-solid form. A tablet may comprise a solid carrier, such as gelatin or an adjuvant. Formulations for rectal or vaginal administration may be presented as a suppository with a suitable carrier; see also O'Hagan et al., Nature Reviews, Drug Discovery 2(9) (2003), 727-735. Further guidance regarding formulations that are suitable for various types of administration can be found in Remington's Pharmaceutical Sciences, Mace Publishing Company, Philadelphia, PA, 17th ed. (1985) and corresponding updates. For a brief review of methods for drug delivery see Langer, Science 249 (1990), 1527-1533.

Unmodified, naked antisense molecules were reported to be internalized poorly by cells, whether or not they are negatively charged (Grey et al., Biochem. Pharmacol. 53 (1997). 1465-1476, Stein et al., Biochemistry 32 (1993), 4855-4861. Bennet et al., Mol. Pharmacol. 41 (1992), 1023-1033). Therefore, the oligonucleotides may be modified or used in compositions with other agents such as lipid carriers (Fattal et al., Adv. Drug Deliv. Rev. 56 (2004), 931-946), microparticles (Khan et al., J. Drug Target 12 (2004), 393-404) vesicles such as exosomes (see Example 4) or by covalent conjugation to cell-penetrating peptides (CPP) allowing translocation of the antisense molecules through the cell membrane; see Lysik and Wu-Pong, J. Pharm. Sci. 92 (2003), 1559-1573 for an review.

### Exosomes:

In this respect vesicles or exosomes may be used as well. "Exosomes" are vesicles of endosomal origin of about 30-100 nm that are secreted in the extracellular milieu following fusion of late endosomal multivesicular bodies with the plasma membrane (Garin et al., J Cell Biol 152 (2001), 165-180). Cells from various tissue types have been shown to secrete exosomes, such as dendritic cells, B lymphocytes, tumor cells and mast cells, for instance. Exosomes from different origin exhibit discrete sets of proteins and lipid moieties (J Thery et al., Cell Biol 147 (1999), 599-610; Thery et al., J Immunol 166 (2001), 7309-7318). They notably contain proteins involved in antigen presentation and immunomodulation suggesting that exosomes play a role in cell-cell communications (Simons and Raposo, Curr. Opin. Cell Biol. 21 (2009), 575-581; Thery et al., Nat. Rev. Immunol. 9 (2009), 581-593) leading to the modulation of immune responses. Methods of producing, purifying or using exosomes for therapeutic purposes or as research tools have been described for instance in WO99/03499, WO00/44389 and WO97/05900, the disclosure content of which is incorporated herein by reference. Furthermore, methods for producing artificial exosomes are known in the art as well. Such artificial endosomes can be derived from coated liposomes as described in De La Peña et al., J Immunol Methods. 344 (2009), 121-132.

Considering their immunogenic and therapeutic properties, it would be particularly useful to be able to modify the content of exosomes in order to alter their properties. In this respect, recombinant exosomes have been described in the art, which derive from cells transfected with plasmids encoding recombinant proteins. Such recombinant exosomes contain the plasmid-encoded recombinant protein (WO00/28001).

### Expression:

The term "expression" as used herein refers to a process by which a gene produces a biochemical, for example, a RNA, a miRNA or polypeptide. The process includes any manifestation of the functional presence of the gene within the cell including, without limitation, gene knockdown as well as both transient expression and stable expression. It includes without limitation transcription of the gene into messenger RNA (mRNA), transfer RNA (tRNA), small hairpin RNA (shRNA), small interfering RNA (siRNA) or any other RNA product, and the translation of such mRNA into polypeptide(s). If the final desired product is a biochemical, expression includes the creation of that biochemical and any precursors. Expression of a gene produces a "gene product." As used herein, a gene product can be either a nucleic acid, *e.g.,* micro RNA (miRNA), a messenger RNA produced by transcription of a gene, or a polypeptide which is translated from a transcript. Gene products described herein further include nucleic acids with post transcriptional modifications, e.g., polyadenylation, or polypeptides with post translational modifications, e.g., methylation, glycosylation, the addition of lipids, association with other protein subunits, proteolytic cleavage, and the like.

A variety of expression vector/host systems may be utilized to contain and express polynucleotide sequences. These include, but are not limited to, microorganisms such as bacteria transformed with recombinant bacteriophage, plasmid, or cosmid DNA expression vectors; yeast transformed with yeast expression vectors; insect cell systems infected with virus expression vectors (e.g., baculovirus); plant cell systems transformed with virus expression vectors (e.g., cauliflower mosaic virus, CaMV; tobacco mosaic virus, TMV) or with bacterial expression vectors (e.g., Ti or pBR322 plasmids); animal or human cell systems.

To express the miRNA, peptide, polypeptide or fusion protein (hereinafter referred to as "product") in a host cell, a procedure such as the following can be used. A restriction fragment containing a DNA sequence that encodes said product may be cloned into an appropriate recombinant plasmid containing an origin of replication that functions in the host cell and an appropriate selectable marker. The plasmid may include a promoter for inducible expression of the product (e.g., pTrc (Amann et al, Gene 69 (1988), 301 315) and pETl Id (Studier et al., Gene Expression Technology: Methods in Enzymology 185, Academic Press, San Diego, Calif. (1990), 60 89). The recombinant plasmid may be introduced into the host cell by, for example, electroporation and cells containing the recombinant plasmid may be identified by selection for the marker on the plasmid. Expression of the product may be induced and detected in the host cell using an assay specific for the product.

In some embodiments, the DNA that encodes the product/miRNA/polypeptide may be optimized for expression in the host cell. For example, the DNA may include codons for one or more amino acids that are predominant in the host cell relative to other codons for the same amino acid.

An example of inducible promoters is the combination of minimal promoters, such as the CMV promoter without the upstream enhancer sequence (sequence from +75 to -53 from the original CMV promoter; see Gossen and Bujard, Proc Natl Acad Sci U S A. 89 (1992), 5547-5551) with tetracycline-resistance operon sequences promoter, which shows a concentration dependent activity the presence of various Tetracycline/Doxycycline concentrations. The non-modified CMV promoter may be used for constitutive expression.

Use of an inducible promoter may lead to a reduced cytotoxicity of RNA accumulation or over-saturation in the miRNA expressing cells. Alternatively, according to the present invention a constitutive expression system capable of consistently expressing the intended miRNAs effectors for a certain period of time may be used. Preferably, the expression of miRNAs or their analogues is driven by a CMV promoter, which is often silenced after about one-month activation in human cells due to DNA methylation. Such a one-month activation mechanism may be beneficial by preventing RNA accumulation or over-saturation in the treated cells.

Delivery of the miRNA expressing nucleic acid composition into human cells can be accomplished using a non-transgenic or transgenic method selected from the group of liposomal/polysomal/chemical transfection, DNA recombination, electroporation, gene gun penetration, transposon/retrotransposon insertion, jumping gene integration, micro-injection, viral infection, retroviral/lentiviral infection, and a combination thereof. To prevent the risks of random transgene insertion and cell mutation liposomal or polysomal transfection may be used to deliver the miRNA sequence comprising vector into the targeted human cells (e.g., the patient's autologous cells). In a particularly preferred embodiment, artificial exosomes may be used; see also Example 4 in this respect. The expression of the chosen, at least one miRNA, a precursor, variant or analogue thereof is dependent on the chosen promoter and may be, e.g., constitutive, inducible or temporarily.

The present invention also relates to kits comprising a nucleic acid of the invention together with any or all of the following: assay reagents, buffers, probes and/or primers, and sterile saline or another pharmaceutically acceptable carrier. In addition, the kits may include instructional materials containing directions (e.g., protocols) for the practice of the methods of this invention.

These and other embodiments are disclosed and encompassed by the description and examples of the present invention. Further literature concerning any one of the materials, methods, uses and compounds to be employed in accordance with the present invention may be retrieved from public libraries and databases, using for example electronic devices. For example the public database "Medline" may be utilized, which is hosted by the National Center for Biotechnology Information and/or the National Library of Medicine at the National Institutes of Health. Further databases and web addresses, such as those of the European Bioinformatics Institute (EBI), which is part of the European Molecular Biology Laboratory (EMBL) are known to the person skilled in the art and can also be obtained using internet search engines. An overview of patent information in biotechnology and a survey of relevant sources of patent information useful for retrospective searching and for current awareness is given in Berks, TIBTECH 12 (1994), 352-364.

The above disclosure generally describes the present invention. Unless otherwise stated, a term as used herein is given the definition as provided in the Oxford Dictionary of Biochemistry and Molecular Biology, Oxford University Press, 1997, revised 2000 and reprinted 2003, ISBN 0 19 850673 2. Several documents are cited throughout the text of this specification. Full bibliographic citations may be found at the end of the specification immediately preceding the claims. The contents of all cited references (including literature references, issued patents, published patent applications as cited throughout this application and manufacturer's specifications, instructions, etc.) are hereby expressly incorporated by reference; however, there is no admission that any document cited is indeed prior art as to the present invention.

A more complete understanding can be obtained by reference to the following specific examples which are provided herein for purposes of illustration only and are not intended to limit the scope of the invention.

### Examples

The examples which follow further illustrate the invention, but should not be construed to limit the scope of the invention in any way. The following experiments in Examples 1 to 12 are illustrated and described with respect to the miRNA-gene clusters, the individual miRNA genes as identified in the placental samples, and new uses of these miRNAs, their precursors, variants and analogues in the treatment and diagnosis of several diseases associated with deregulated immune response, for example during pregnancy; see also the Figures and the Tables 1 to 3 in this respect.

### Example 1: Relative expression of miR-520c-3p in placental tissue in relation to the week of gestation

### Materials and Methods

### RNA Isolation

Total RNA was isolated from 52 formalin fixed and paraffin embedded (FFPE) placenta tissues from spontaneous and induced abortions occurring between the 7th and 33rd week of gestation. RNA isolation was performed using the innuPREP Micro RNA Kit (Analytik Jena AG, Jena, Germany) according to the manufacturer's instructions with the following modification for FFPE tissues: lysis of the paraffin sections was conducted by incubating the sections in TLS-Lysis Solution and Proteinase K from the innuPREP DNA Micro Kit (Analytik Jena) for 1h at 60°C and 15 min at 80°C.

### Reverse Transcription and Real-time PCR

200 ng of total RNA were used to generate cDNA specific to miR-520c and RNU6B (which served as an internal control for relative quantification; CGCAAGGATGACACGCAAATTCGTGAAGCGTTCCATATTTTT SEQ ID NO 79) with the TaqMan microRNA Reverse Transcription Kit and RT primers from the TaqMan microRNA Assays (Applied Biosystems, Foster City, CA, USA) according to the manufacturer's instructions. The reactions were incubated in a thermal cycler for 30 min at 16°C, 30 min at 42°C, and 5 min at 85°C. Real-time PCR reactions were set up using miRNA specific probes and primers included in the TaqMan microRNA Assays and the TaqMan Universal PCR Master Mix (Applied Biosystems, Foster City, CA, USA). The reactions were incubated in 96-well plates at 95°C for 10 min followed by 40 cycles of 15 s at 95°C and one minute at 60°C. All reactions were run in triplicate on an Applied Biosystems 7300 Fast Real Time PCR system. Relative quantification (RQ) was calculated using the ΔΔCt method (Livak and Schmittgen, 2001). *RNU6B* served as endogenous control for normalization.

### Results

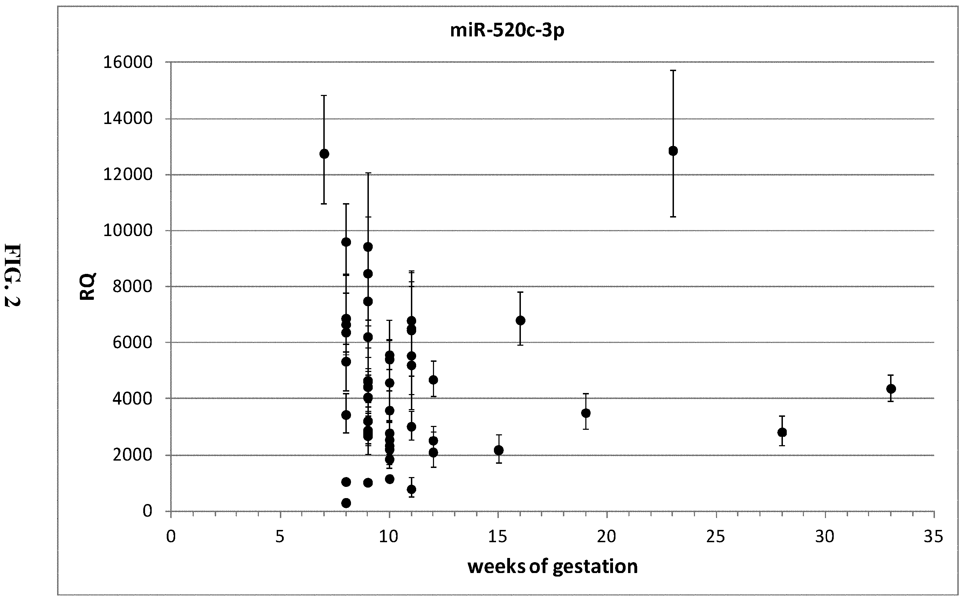

The relative expression of miR-520c-3p was plotted against the week of gestation (Fig. 2). The data indicates a slight decrease of the expression of miR-520c-3p with advancing pregnancy which is in contrast to Luo et al. (2009) who stated an increase of the expression of chromosome 19 miRNAs with advancing pregnancy.

### References

Livak, K. J. and T. D. Schmittgen (2001). "Analysis of relative gene expression data using real-time quantitative PCR and the 2(-Delta Delta C(T)) Method." Methods 25(4): 402-408.
Luo, S. S., O. Ishibashi, et al. (2009). "Human villous trophoblasts express and secrete placenta-specific microRNAs into maternal circulation via exosomes." Biol Reprod 81(4): 717-729.

### Example 2: Correlation of the relative expression of miR-371-3p, miR-372, and miR-373 with miR-520c-3p in placental tissue

### Materials and Methods

### RNA Isolation

Total RNA was isolated from 52 formalin fixed and paraffin embedded (FFPE) placental tissues from spontaneous and induced abortions. RNA isolation was performed using the innuPREP Micro RNA Kit (Analytik Jena AG, Jena, Germany) according to the manufacturer's instructions with the following modification for FFPE tissues: lysis of the paraffin sections was conducted by incubating the sections in TLS-Lysis Solution and Proteinase K from the innuPREP DNA Micro Kit (Analytik Jena) for 1h at 60°C and 15 min at 80°C.

### Reverse Transcription and Real-time PCR

200 ng of total RNA were used to generate cDNA specific to miR-520c, miR-371-3p, miR-372, miR-373, and RNU6B (which served as an internal control for relative quantification) with the TaqMan microRNA Reverse Transcription Kit and RT primers from the TaqMan microRNA Assays (Applied Biosystems, Foster City, CA, USA) according to the manufacturer's instructions. The reactions were incubated in a thermal cycler for 30 min at 16°C, 30 min at 42°C, and 5 min at 85°C. Real-time PCR reactions were set up using miRNA specific probes and primers included in the TaqMan microRNA Assays and the TaqMan Universal PCR Master Mix (Applied Biosystems, Foster City, CA, USA). The reactions were incubated in 96-well plates at 95°C for 10 min followed by 40 cycles of 15 s at 95°C and one minute at 60°C. All reactions were run in triplicate on an Applied Biosystems 7300 Fast Real Time PCR system. Relative quantification (RQ) was calculated using the ΔΔCt method (Livak and Schmittgen, 2001). *RNU6B* served as endogenous control for normalization.

### Statistical analysis

Regression analysis of the expression data was performed using a linear regression t-test.

### Results

The regression analysis results are summarized in Table 3. The relative expression of miR-371-3p, miR-372, and miR-373 correlated highly significantly with the expression of miR-520c-3p, respectively. This correlation indicates a coordinated expression of these miRNAs and thus suggests that they share similar functions.

**Table 3: Highly significant correlation between the expression of miR-371-3p, miR-372, and miR-373, and miR-520c-3p.**

| | miR-520c-3p |
|---|---|
| miR-371-3p | p<0,001 |
| miR-372 | p<0,001 |
| miR-373 | p<0,001 |

### References

Livak, K. J. and T. D. Schmittgen (2001). "Analysis of relative gene expression data using real-time quantitative PCR and the 2(-Delta Delta C(T)) Method." Methods 25(4): 402-408.

### Example 3: Comparison of the relative expression of miR-371-3p, miR-372, miR-373, and miR-520c-3p in stromal and trophoblast cells

### Materials and Methods

### Microdissection

One of formalin fixed and paraffin embedded sample of an apparently normal first trimester placenta (8th week of gestation) was used for the separate analysis of stromal and trophoblast compartment.

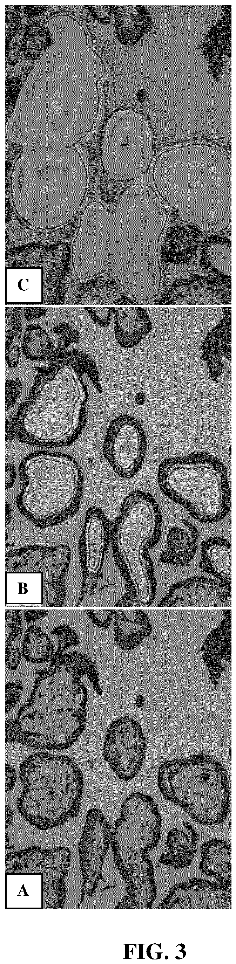

Using standard procedures for laser microdissection as described by the manufacturer (http://www.leica-microsystems.com/products/light-microscopes/life-science-research/laser-microdissection/details/product/leica-lmd7000/downloads/ accessed on Nov 17, 2011) the stromal core and the trophoblast layer were separated (Fig. 3). In this respect laser microdissection can be also performed as described in Grundemann et al. Nucleic Acids Res 36 (2008), e38, in particular at page 3 in the section "UV-Laser-microdissection and cDNA synthesis of microdissected cells", the disclosure content of which is herein incorporated by reference.

### RNA Isolation

Total RNA was isolated from the stromal and the trophoblast cells using QIAGEN miRNEasy Mini Kit (QIAGEN, Hilden, Germany) according to the manufacturer's instructions.

### Reverse Transcription and Real-time PCR

10 ng of total RNA were used to generate cDNA specific to miR-520c, miR-371-3p, miR-372, miR-373, and RNU6B (which served as an internal control for relative quantification) with the TaqMan microRNA Reverse Transcription Kit and RT primers from the TaqMan microRNA Assays (Applied Biosystems, Foster City, CA, USA) according to the manufacturer's instructions. The reactions were incubated in a thermal cycler for 30 min at 16°C, 30 min at 42°C, and 5 min at 85°C. Real-time PCR reactions were set up using miRNA specific probes and primers included in the TaqMan microRNA Assays and the TaqMan Universal PCR Master Mix (Applied Biosystems, Foster City, CA, USA). The reactions were incubated in 96-well plates at 95°C for 10 min followed by 40 cycles of 15 s at 95°C and one minute at 60°C. All reactions were run in triplicate on an Applied Biosystems 7300 Fast Real Time PCR system. Relative quantification (RQ) was calculated using the ΔΔCt method (Livak and Schmittgen, 2001). *RNU6B* served as endogenous control for normalization.

### Results

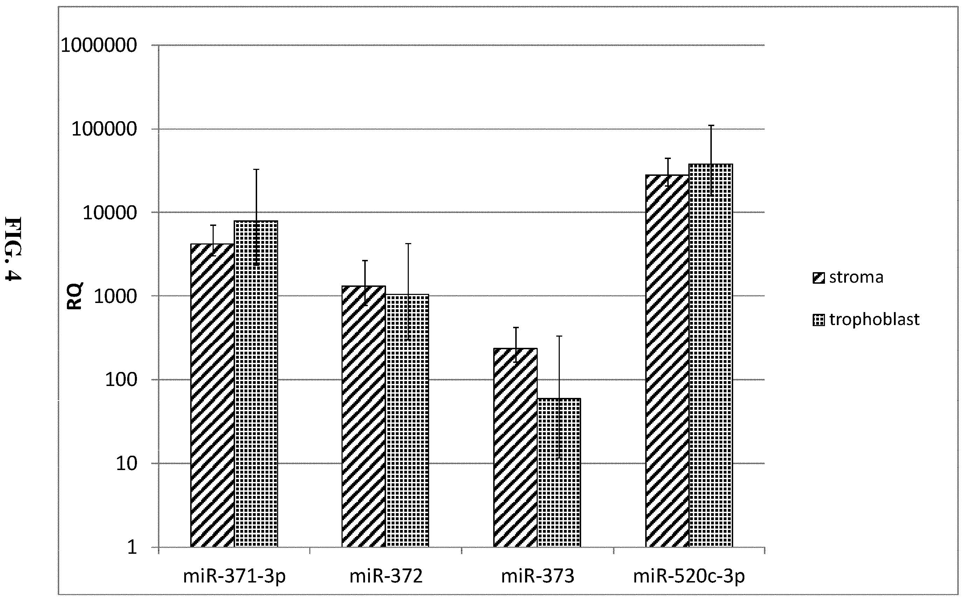

In contrast to results formerly obtained by Luo et al., (2009) who detected C19MC miRNAs only in the trophoblast layer and not in stromal cells the results presented herein show that the expression of miR-520c-3p is comparably high in the stromal cells as in the trophoblast cells. The same holds true for the expression levels of miR-371-3p and miR-372. miR-373 is even higher expressed in stromal cells than in trophoblast cells (Figure 4).

### Example 4: Test of the immunomodulatory properties of the miRNAs and binding molecules of the present invention.

The ability of the microRNAs or binding molecules of the present invention as defined herein above to suppress the immune system is tested by transfecting mesenchymal stem cells or trophoblast cells with microRNAs and isolation of exosomes released by these cells for use in experiments aimed at the immunomodulation of target cells of these exosomes. Methods for extraction of exosomes are known in the art and used as described, e.g., in Hedlund et al. (2009), in Materials and Methods part, in particular at pages 342 to 343 in the section "Isolation of exosomes from supernatants of placental explant cultures", or as described in Taylor et al. (2006) at page 1535 in the section "Isolation of circulating exosomes" the disclosure content of both is incorporated herein by reference. Alternatively, the microRNAs is used directly to transfect the target cells which can, e.g., be lymphocytes or dendritic cells following routine methods as outlined, e.g., by Marasa et al. (2010) at page 340 in the section "Cell culture, transfections and b-galactosidase staining" analogously for HeLa cells and fibroblasts, the disclosure content of which is incorporated herein by reference. There are several ways to test the immunomodulatory ability of the microRNAs on the level of the recipient cells. The relevant target cells, methods of transfection and the parameters used to evaluate the microRNAs potential to suppress the immune system are known in the art and used as described, e.g., by Sabapatha et al., 2006; Taylor et al., 2006; Hegmans et al., 2008; Hedlund et al., 2009; Ren et al., 2011; Zhang et al., 2011, in particular in the respective Materials and Methods sections, the disclosure content of which is incorporated herein by reference.

### Example 5: Using miRNA mimics to suppress T-cell activity

Electroporation was used to transfect T-cells and T-cell derived cell lines with mimics of miRNAs of C19MC (Qiagen, Hilden, Germany) at appropriate concentrations to test their proliferative capacity and cytokine expression. As a negative control scrambled siRNAs (Qiagen) are used. Efficiency of transfection is tested using AllStars Hs Cell Death siRNA (Qiagen).

### Example 6: Relative expression of miR-517a-3p, miR-519a-3p, and miR-520c-3p in term placenta and amniotic membrane tissue

Mesenchymal cells from the amniotic membrane appear to have strong immunomodulatory properties, e.g., by actively suppressing T-cell proliferation induced by alloantigenes (Wolbank et al., 2007). Thus, the expression of miR-517a-3p, miR-519a-3p, and miR-520c-3p was measured in a term amniotic membrane and compared to the expression levels of the corresponding placenta tissue.

### Materials and Methods

### RNA Isolation

Total RNA was isolated from formalin fixed and paraffin embedded (FFPE) term placenta tissue and adjacent amniotic membrane tissue obtained shortly after delivery. RNA isolation was performed using the innuPREP Micro RNA Kit (Analytik Jena AG, Jena, Germany) according to the manufacturer's instructions with the following modification for FFPE tissues: lysis of the paraffin sections was conducted by incubating the sections in TLS-Lysis Solution and Proteinase K from the innuPREP DNA Micro Kit (Analytik Jena) for 1h at 60°C and 15 min at 80°C.

### Reverse Transcription and Real-time PCR

200 ng of total RNA were used to generate cDNA specific to miR-517a-3p, miR-519a-3p, miR-520c-3p and RNU6B (which served as an internal control for relative quantification) with the TaqMan microRNA Reverse Transcription Kit and RT primers from the TaqMan microRNA Assays (Applied Biosystems, Foster City, CA, USA) according to the manufacturer's instructions. The reactions were incubated in a thermal cycler for 30 min at 16°C, 30 min at 42°C, and 5 min at 85°C. Real-time PCR reactions were set up using miRNA specific probes and primers included in the TaqMan microRNA Assays and the TaqMan Universal PCR Master Mix (Applied Biosystems, Foster City, CA, USA). The reactions were incubated in 96-well plates at 95°C for 10 min followed by 40 cycles of 15 s at 95°C and one min at 60°C. All reactions were run in triplicate on an Applied Biosystems 7300 Fast Real Time PCR system. Relative quantification (RQ) was calculated using the ΔΔCt method (Livak und Schmittgen, 2001). *RNU6B* served as endogenous control for normalization.

### Results

The relative expression of miR-517a-3p, miR-519a-3p, and miR-520c-3p was measured in term amniotic membrane tissue and compared to corresponding placenta tissue. The obtained RQ (relative quantification) values are given in Table 4, below.

**Table 4: Relative quantification (RQ) values of miR-517a-3p, miR-519a-3p, and miR-520c-3p obtained in placenta and amniotic membrane tissue.**

| | miR-517a-3p | miR-519a-3p | miR-520c-3p |
|---|---|---|---|
| | RQ (RQ range) | RQ (RQ range) | RQ (RQ range) |
| term placenta | 1 (0.954 - 1.048) | 1 (0.911 - 1.098) | 1 (0.859 - 1.165) |
| term amniotic membrane | 0.522 (0.456 - 0.597) | 0.810 (0.692 -0.949) | 0.731 (0.633 - 0.844) |

These data show that the expression of miR-517a-3p, miR-519a-3p, and miR-520c-3p is comparably high in amniotic membrane as in placenta tissue.

### References:

Hedlund, M., A. C. Stenqvist, et al. (2009). "Human placenta expresses and secretes NKG2D ligands via exosomes that down-modulate the cognate receptor expression: evidence for immunosuppressive function." J Immunol 183(1): 340-351.
Hegmans, J. P., P. J. Gerber, et al. (2008). "Exosomes." Methods Mol Biol 484: 97-109.
Livak, K. J. and T. D. Schmittgen (2001). "Analysis of relative gene expression data using real-time quantitative PCR and the 2(-Delta Delta C(T)) Method." Methods 25(4): 402-408.
Marasa, B. S., S. Srikantan, et al. (2010). "MicroRNA profiling in human diploid fibroblasts uncovers miR-519 role in replicative senescence." Aging (Albany NY) 2(6): 333-343.
Ren, Y., J. Yang, et al. (2011). "Exosomal-like vesicles with immune-modulatory features are present in human plasma and can induce CD4+ T-cell apoptosis in vitro." Transfusion 51(5): 1002-1011.
Sabapatha, A., C. Gercel-Taylor, et al. (2006). "Specific isolation of placenta-derived exosomes from the circulation of pregnant women and their immunoregulatory consequences." Am J Reprod Immunol 56(5-6): 345-355.
Taylor, D. D., S. Akyol, et al. (2006). "Pregnancy-associated exosomes and their modulation of T cell signaling." J Immunol 176(3): 1534-1542.
Wolbank, S., A. Peterbauer, et al. (2007). "Dose-dependent immunomodulatory effect of human stem cells from amniotic membrane: a comparison with human mesenchymal stem cells from adipose tissue." Tissue Engineering 13(6): 1173-1183.
Zhang, H., Y. Xie, et al. (2011). "CD4(+) T cell-released exosomes inhibit CD8(+) cytotoxic T-lymphocyte responses and antitumor immunity." Cell Mol Immunol 8(1): 23-30.

### Example 7: Comparison of the relative expression of miR-520c-3p and miR-517a-3p in decidua and trophoblast cells

### Materials and methods

### Microdissection

A formalin fixed and paraffin embedded sample of an apparently normal first trimester placenta (9th week of gestation) was used for the separate analysis of trophoblast and deciduas.

Using standard procedures for laser microdissection as described by the manufacturer (http://www.leica-microsystems.com/products/light-microscopes/life-science-research/laser-microdissection/details/product/leica-lmd7000/downloads/ accessed on Nov 17, 2011; or the "Materials and methods" section, subsection "Laser microdissection" in the left column on page 303 of Asztalos et al., (2010), the disclosure content of which is herein incorporated by reference.) the trophoblast and decidua tissue were separated. the stromal core and the trophoblast layer were separated (Fig. 3). In this respect laser microdissection can be also performed as described in Grundemann et al. Nucleic Acids Res 36 (2008), e38, in particular at page 3 in the section "UV-Laser-microdissection and cDNA synthesis of microdissected cells", the disclosure content of which is herein incorporated by reference.

### RNA Isolation

Total RNA was isolated from the decidual and the trophoblast cells using QIAGEN miRNEasy Mini Kit (QIAGEN, Hilden, Germany) according to the manufacturer's instructions.

### Reverse Transcription and Real-time PCR

10 ng of total RNA were used to generate cDNA specific to miR-520c-3p, miR-517a-3p and RNU6B (which served as an internal control for relative quantification) with the TaqMan microRNA Reverse Transcription Kit and RT primers from the TaqMan microRNA Assays (Applied Biosystems, Foster City, CA, USA) according to the manufacturer's instructions. The reactions were incubated in a thermal cycler for 30 min at 16°C, 30 min at 42°C, and 5 min at 85°C. Real-time PCR reactions were set up using miRNA specific probes and primers included in the TaqMan microRNA Assays and the TaqMan Universal PCR Master Mix (Applied Biosystems, Foster City, CA, USA). The reactions were incubated in 96-well plates at 95°C for 10 min followed by 40 cycles of 15 s at 95°C and one min at 60°C. All reactions were run in triplicate on an Applied Biosystems 7300 Fast Real Time PCR system. Relative quantification (RQ) was calculated using the ΔΔCt method (Livak und Schmittgen, 2001). *RNU6B* served as endogenous control for normalization. The expression was compared to a thyroid tumor expressing miR-520c-3p and miR-517a-3p at low levels.

### Results

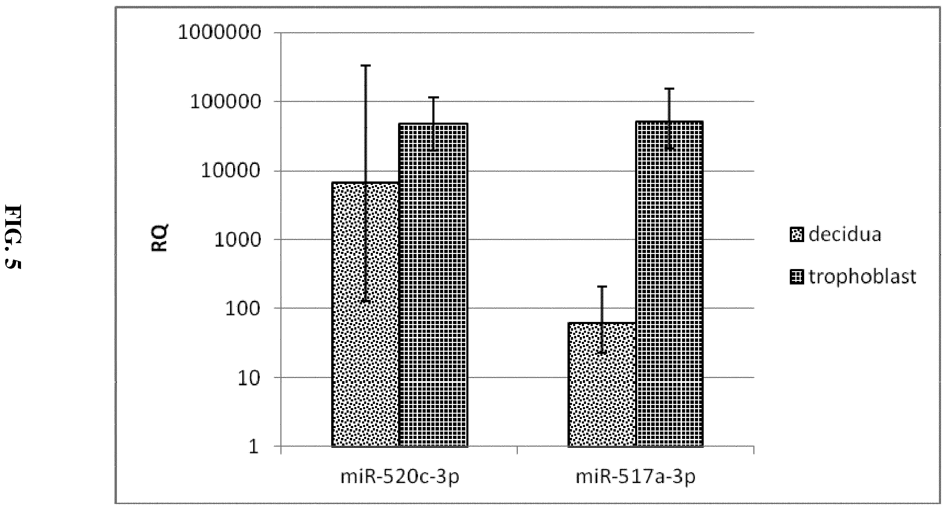

The results presented herein show that miR-520c-3p and miR-517a-3p are not only present in trophoblast cells but also in decidual cells (Figure 5). The decidua consists of maternal cells with a maternal methylation pattern that do not express C19MC microRNAs. Data presented herein therefore show that the presence of miR-517a-3p and miR-520c-3p cells in this tissue is due to the uptake of miRNAs (originally released via exosomes by placental cells) by decidual cells or, more specifically, decidual immune cells.

### Example 8: In silico analysis of C19MC microRNAs, their validated targets and potential role in immunomodulation

### Materials and methods

The microRNA registry miRBase (http://www.mirbase.org/) was searched for validated targets of microRNAs of C19MC. The relevant literature was then searched for known functions of these validated targets.

### Results

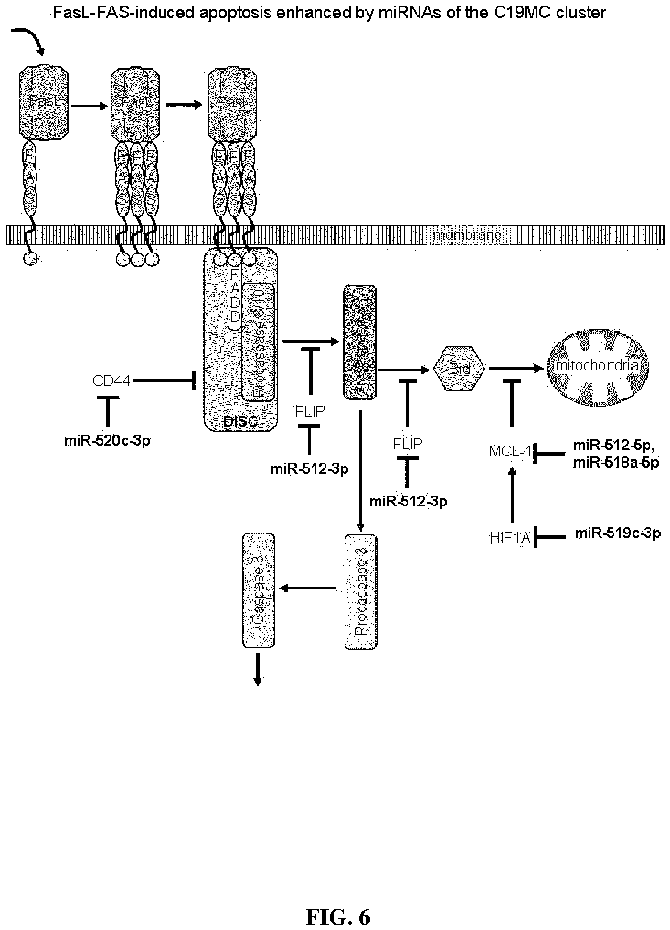

The results presented herein show that high proportion of the microRNAs of C19MC target genes is associated with apoptosis and immunomodulation. As an example there are validated targets that act as inhibitors of Fas - FasL induced apoptosis. These targets and the corresponding miRNAs of the C19MC cluster are schematically shown in Fig. 6.

### References

"miRBase (Release 18)." from http://www.mirbase.org.
Asztalos S, Gann PH, Hayes MK, Nonn L, Beam CA, Dai Y, Wiley EL, Tonetti DA: "Gene expression patterns in the human breast after pregnancy" Cancer Prev Res (Phila). 2010 Mar;3(3):301-11
Bentwich I, Avniel A, Karov Y, Aharonov R, Gilad S, Barad O, Barzilai A, Einat P, Einav U, Meiri E, Sharon E, Spector Y, Bentwich Z (2005). "Identification of hundreds of conserved and nonconserved human microRNAs" Nat Genet. 37:766-770.
Griffiths-Jones S, Grocock RJ, van Dongen S, Bateman A, Enright AJ. (2006). "miRBase: microRNA sequences, targets and gene nomenclature." Nucl. Acids Res. 34 (suppl 1) (Database Issue):D140-D144
Griffiths-Jones S. (2004). "The microRNA Registry." Nucl. Acids Res. 32 (suppl 1) (Database Issue):D 109-D 111
Griffiths-Jones S, Saini HK, van Dongen S, Enright AJ. (2008). "miRBase: tools for microRNA genomics." Nucl. Acids Res. 36 (suppl 1) (Database Issue):D154-D158
Kozomara A, Griffiths-Jones S. (2011). "miRBase: integrating microRNA annotation and deep-sequencing data." Nucl. Acids Res. 39 (suppl 1) (Database Issue):D152-D157
Landgraf P, Rusu M, Sheridan R, Sewer A, Iovino N, Aravin A, Pfeffer S, Rice A, Kamphorst AO, Landthaler M, Lin C, Socci ND, Hermida L, Fulci V, Chiaretti S, Foa R, Schliwka J, Fuchs U, Novosel A, Muller RU, Schermer B, Bissels U, Inman J, Phan Q, Chien M (2007). "A mammalian microRNA expression atlas based on small RNA library sequencing" Cell. 129:1401-1414.
Livak, K. J. and T. D. Schmittgen (2001). "Analysis of relative gene expression data using real-time quantitative PCR and the 2(-Delta Delta C(T)) Method." Methods 25(4): 402-408.
Luo, S. S., O. Ishibashi, et al. (2009). "Human villous trophoblasts express and secrete placenta-specific microRNAs into maternal circulation via exosomes." Biol Reprod 81(4): 717-729.
Pandolfi F, Cianci R, Pagliari D, Casciano F, Bagalà C, Astone A, Landolfi R, Barone C. "The immune response to tumors as a tool toward immunotherapy." Clin Dev Immunol.; 2011:894704. Epub 2011 Dec 5.
Roelen, D. L., B. J. van der Mast, et al. (2009). "Differential immunomodulatory effects of fetal versus maternal multipotent stromal cells." Hum Immunol 70(1): 16-23.
Southcombe, J., D. Tannetta, et al. (2011). "The immunomodulatory role of syncytiotrophoblast microvesicles." PLoS One 6(5): e20245.
Valadi, H., K. Ekstrom, et al. (2007). "Exosome-mediated transfer of mRNAs and microRNAs is a novel mechanism of genetic exchange between cells." Nat Cell Biol 9(6): 654-659.
Warning, J. C., S. A. McCracken, et al. (2011). "A balancing act: mechanisms by which the fetus avoids rejection by the maternal immune system." Reproduction 141(6): 715-724.

### Example 9: Cells from the bovine amniotic membrane fail to inhibit the mixed lymphocyte reaction

Human amniotic membrane cells are known to induce an inhibitory effect on mixed lymphocyte reactions (MLR) (Magatti et al., 2008). Experiments provided within the present invetnion demonstrate that these cells also abundantly express C19MC microRNAs (see Example 6). Bovine amniotic membrane cells (bAMC) which are not expressing C19MC microRNAs should exert no inhibitory effect or a less pronounced one. Thus, MLR was performed in presence of bAMC and proliferation of the PBMCs (peripheral blood mononuclear cells) was measured by BrdU assay.

### Materials and Methods

### Cell cultures

Bovine amniotic membrane-derived cells (bAMC) were cocultured in RPMI complete medium with either
- PBMC from one donor (donor A)
- PBMC from another donor (donor B)
- PBMCs from both donors (donor A + donor B) (mixed lymphocyte reaction (MLR))
- PBMC from donor A + T-cell stimulant conA (concanavalin A); or
- PBMC from donor B + T-cell stimulant conA

As a positive control the same experimental setup was performed with JEG-3 cells instead of bAMC. The choriocarcinoma-derived cell line JEG-3 abundantly expresses C19MC microRNAs (Morales-Prieto et al., 2012). Furthermore, this cell line is known to exert an inhibitory effect on T cells (Hammer et al., 2002). The cell line S40.2 is derived from a thyroid adenoma and known to overexpress the microRNAs of the clusters C19MC and miR-371-373 (Rippe et al., 2010).

JEG-3 and bAMC were irrradiated with 3000 Gy to ensure that the proliferation observed could be attributed to the lymphocytes. To control for the stimulatory potential of the PBMCs, a mixed lymphocyte reaction (MLR) was set up using PBMCs from the two different donors A and B without the addition of bAMC or JEG-3 cells. All experiments were performed for 96h and 120h and all cultures were carried out in triplicate.

### BrdU assay

24h before the end of the experiments, BrdU was added to the cell cultures. After 96h and 72h, respectively, the supernatant (containing the PBMCs in suspension) was collected and the PBMCs' proliferation was measured by BrdU assay (Roche Applied Science, Mannheim, Germany).

### Results

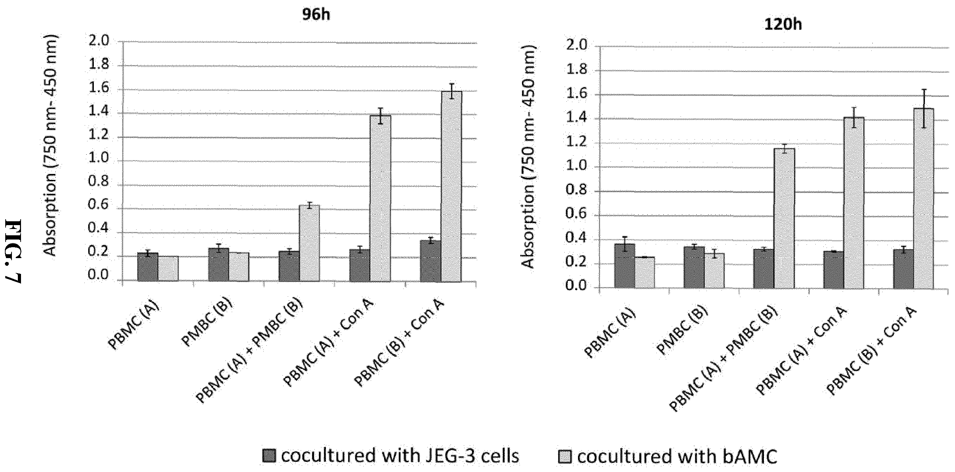

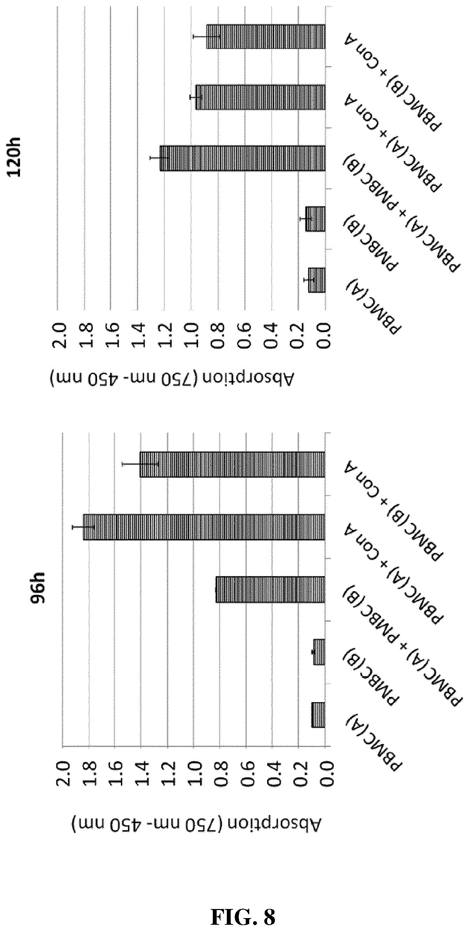

The bAMCs failed to induce an inhibitory effect on the MLR or the PBMC stimulated with Con A whereas the JEG-3 cells effectively suppressed PBMC activation (Fig. 7). PBMCs used within the present invention were sufficiently activated in MLR and by stimulation with Con A (Fig. 8). Likewise, cells of the thyroid adenoma cell line S40.2 can be used instead of JEG-3 cells to demonstrate their ability to suppress the mixed lymphocyte reaction.

### References:

Hammer, A., M. Hartmann, et al. (2002). "Expression of functional Fas ligand in choriocarcinoma." American Journal of Reproductive Immunology 48(4): 226-234.
Magatti, M., S. De Munari, et al. (2008). "Human amnion mesenchyme harbors cells with allogeneic T-cell suppression and stimulation capabilities." Stem Cells 26(1): 182-192.
Morales-Prieto, D. M., W. Chaiwangyen, et al. (2012). "MicroRNA expression profiles of trophoblastic cells." Placenta.
Rippe, V., L. Dittberner, et al. (2010). "The two stem cell microRNA gene clusters C19MC and miR-371-3 are activated by specific chromosomal rearrangements in a subgroup of thyroid adenomas." PLoS One 5(3): e9485.

### Example 10: A BAC clone containing the cluster C19MC

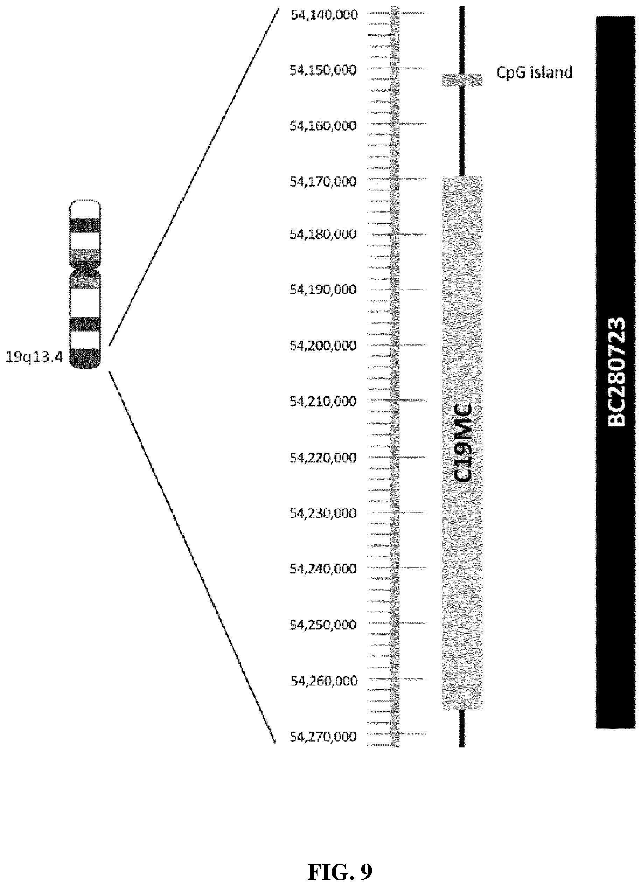

The BAC clone BC280723 (GenBank accession no. Ac011453) spans the entire chromosomal region of C19MC including the adjacent CpG island, i.e. its presumed promoter region (BC280723) (Fig. 9). This sequence is inserted into a pBACe3.6 vector.

### Example 11: Expression of C19MC miRNAs from a BAC clone containing the whole C19MC cluster transfected into cells from the bovine amniotic membrane

### Materials and Methods

### Transfection

Cells cultures derived from bovine amniotic membrane using standard cell cultures techniques/procedures were transfected with a BAC vector containing the cluster C19MC as insert (see Example 10, above). Transfection was performed using QIAGEN's (Hilden, Germany) Attractene Transfection Reagent according to the manufacturer's instructions.

As a negative control cells were mock transfected with a transfection complex not containing BAC vector DNA. Cells were harvested at 24h, 48h and 6 days (144 h) after transfection.

### Reverse Transcription and Real-time PCR

200 ng of total RNA were used to generate cDNA specific to miR-517a-3p and RNU6B (which served as an internal control for relative quantification) with the TaqMan microRNA Reverse Transcription Kit and RT primers from the TaqMan microRNA Assays (Applied Biosystems, Foster City, CA, USA) according to the manufacturer's instructions. The reactions were incubated in a thermal cycler for 30 min at 16°C, 30 min at 42°C, and 5 min at 85°C. Real-time PCR reactions were set up using miRNA specific probes and primers included in the TaqMan microRNA Assays and the TaqMan Universal PCR Master Mix (Applied Biosystems, Foster City, CA, USA). The reactions were incubated in 96-well plates at 95°C for 10 min followed by 40 cycles of 15 s at 95°C and one min at 60°C. All reactions were run in triplicate on an Applied Biosystems 7300 Fast Real Time PCR system. Relative quantification (RQ) was calculated using the ΔΔCt method (Livak and Schmittgen, 2001). RNU6B served as endogenous control for normalization.

### Results

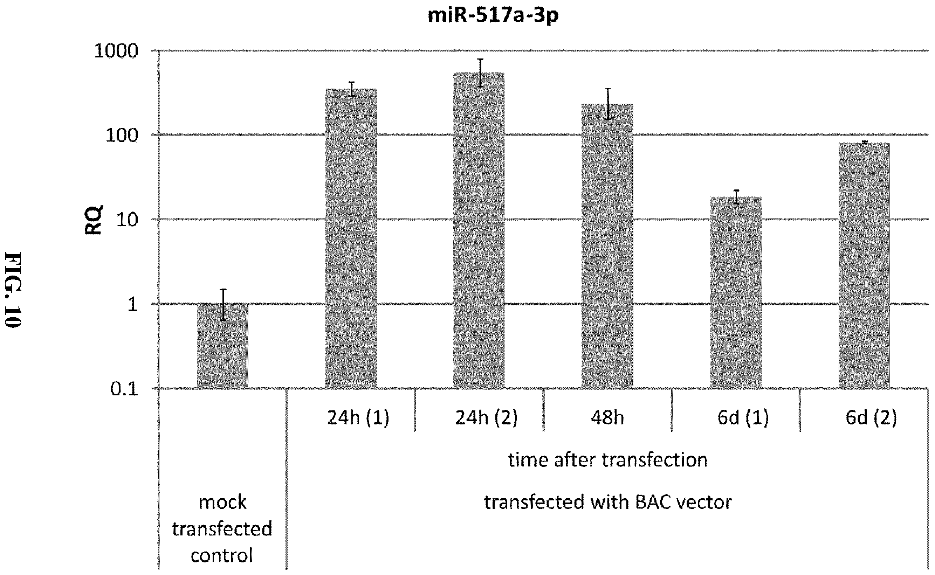

As the microRNA cluster C19MC is primate-specific, it is not expressed in bovine cells. Transfection of bovine amniotic membrane-derived cells with the BAC vector leads to a high expression of miR-517a-3p as compared to mock transfected cells which lasts up to six days at least; see also Fig. 10.

These results indicate that expression of C19MC microRNAs can be attained in cells not expressing these microRNAs and that the available BAC vector is an example of a vector suitable for this purpose.

### Example 12: Downregulation of cFLIP mRNA in Jurkat cells incubated with supernatant from JEG-3 cell cultures

A number of C19MC microRNAs seem to target anti-apoptotic genes involved in Fas-FasL induced apoptosis (see Example 8, above). One of the targets is c-FLIP (CFLAR). C19MC microRNAs are often packed into exosomes which are secreted by the cells. In cell culture these exosomes accumulate in the culture medium.

### Materials and Methods

### Cell culture

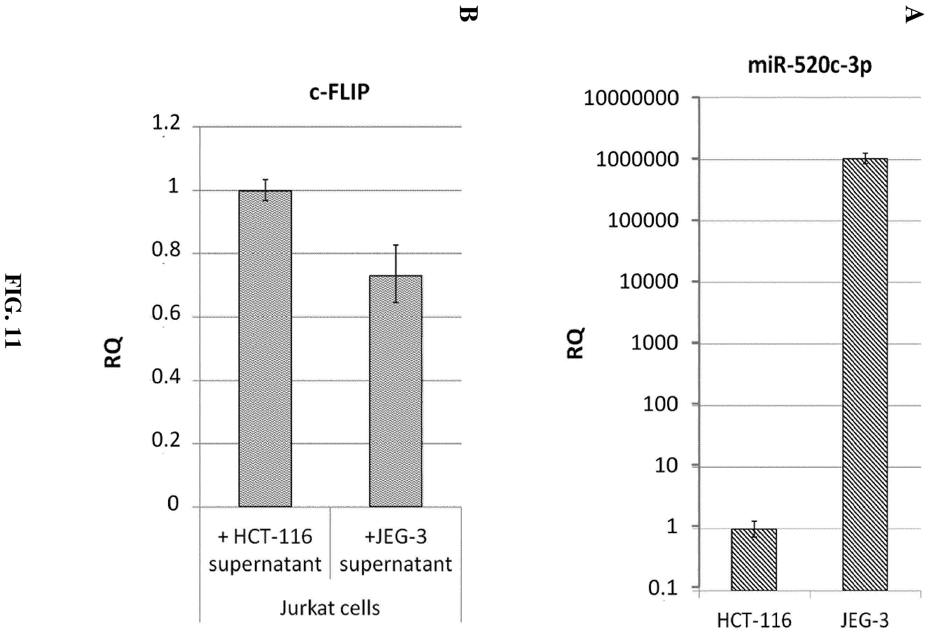

JEG-3 is a choriocarcinoma-derived cell line highly expressing C19MC microRNAs. HCT-116 is a colon carcinoma-derived cell line that does not significantly express C19MC microRNAs (see fig. 11A). Jurkat is a T cell leukemia-derived cell line also not significantly expressing C19MC microRNAs. All three cell lines used are commercially available. All cell lines were grown in RPMI supplemented with 10% fetal calf serum and antibiotics (penicillin/streptomycin).

The supernatants from cell cultures of JEG-3 and HCT-116 cells were collected after three days in culture. 4 ml of the collected JEG-3 supernatant were then added to Jurkat cells grown in 3 ml medium. A Jurkat cell culture incubated with supernatant of HCT-116 cells served as control. Jurkat cells of both preparations were harvested 24h after the addition of the supernatant.

### RNA isolation

Total RNA was isolated from the Jurkat cells using QIAGEN miRNeasy Mini Kit (QIAGEN, Hilden, Germany) according to the manufacturer's instructions.

### Reverse Transcription and Real-time PCR

Reverse transcription was performed with 250 ng of total RNA using M-MLV Reverse Transcriptase (Invitrogen, Karlsruhe, Germany) with 150 ng random hexamers and RNaseOUT^{™} Recombinant Ribonuclease Inhibitor according to the manufacturer's instructions.

Real-time PCR for c-FLIP (CFLAR) was performed using the TaqMan Gene Expression Assay CFLAR (Hs00153439_m1) (Life Technologies, Darmstadt, Germany; Cat # 4331182) and the TaqMan Universal PCR Master Mix (Life Technologies, Darmstadt, Germany) according to the manufacturer's instructions. The reactions were incubated in 96-well plates at 95°C for 10 min followed by 40 cycles of 15 s at 95°C and one min at 60°C. All reactions were run in triplicate on an Applied Biosystems 7300 Fast Real Time PCR system. Relative quantification (RQ) was calculated using the ΔΔCt method (Livak and Schmittgen 2001). HPRT served as endogenous control for normalization.

### Results

Jurkat cells treated with supernatants of JEG-3 cells showed a decreased expression of c-FLIP as compared to Jurkat cells treated with HCT-116 supernatant (Fig. 11B). JEG-3 cells overexpress C19MC miRNAs and the supernatant contains exosomes with these microRNAs which in the present example are delivered to the Jurkat cells. Accordingly, c-FLIP mRNA becomes downregulated.

### References:

Livak, K. J. and T. D. Schmittgen (2001). "Analysis of relative gene expression data using real-time quantitative PCR and the 2(-Delta Delta C(T)) Method." Methods 25(4): 402-408.
Wolbank, S., A. Peterbauer, et al. (2007). "Dose-dependent immunomodulatory effect of human stem cells from amniotic membrane: a comparison with human mesenchymal stem cells from adipose tissue." Tissue Engineering 13(6): 1173-1183.

## Claims

1. A miRNA for use in immunomodulation, wherein the miRNA is selected from the miRNAs encoded by any one of the transcription units comprised in the C19MC cluster or in the miR-371-373 cluster.

2. The microRNA of claim 1, wherein the microRNA is selected from the group consisting of: hsa-miR-498, hsa-miR-512-3p, hsa-miR-512-5p, hsa-miR-515-3p, hsa-miR-515-5p, hsa-miR-516a-3p, hsa-miR-516a-5p, hsa-miR-516b-3p, hsa-miR-516b-5p, hsa-miR-517-5p, hsa-miR-517a-3p, hsa-miR-517b-3p, hsa-miR-517c-3p, hsa-miR-518a-3p, hsa-miR-518a-5p, hsa-miR-518b, hsa-miR-518c-3p, hsa-miR-518c-5p, hsa-miR-518d-3p, hsa-miR-518d-5p, hsa-miR-518e-3p, hsa-miR-518e-5p, hsa-miR-518f-3p, hsa-miR-518f-5p, hsa-miR-519a-3p, hsa-miR-519a-5p, hsa-miR-519b-3p, hsa-miR-519b-5p, hsa-miR-519c-3p, hsa-miR-519c-5p, hsa-miR-519d, hsa-miR-519e-3p, hsa-miR-519e-5p, hsa-miR-520a-3p, hsa-miR-520a-5p, hsa-miR-520b, hsa-miR-520c-3p, hsa-miR-520c-5p, hsa-miR-520d-3p, hsa-miR-520d-5p, hsa-miR-520e, hsa-miR-520f, hsa-miR-520g, hsa-miR-520h, hsa-miR-521, hsa-miR-522-3p, hsa-miR-522-5p, hsa-miR-523-3p, hsa-miR-523-5p, hsa-miR-524-3p, hsa-miR-524-5p, hsa-miR-525-3p, hsa-miR-525-5p, hsa-miR-526a, hsa-miR-526b-3p, hsa-miR-526b-5p, hsa-miR-527, hsa-miR-1283, hsa-miR-1323, hsa-miR-371a-3p, hsa-miR-371a-5p, hsa-miR-371b-3p, hsa-miR-371b-5p, hsa-miR-372, hsa-miR-373-3p, hsa-miR-373-5p having the SEQ ID Nos.: 1 to 66 .

3. A double-stranded nucleic acid comprising the nucleic acid sequence of the miRNA or miRNA precursor of claim 1 or 2.

4. A vector comprising the double-stranded nucleic acid of claim 3.

5. A host cell comprising the vector of claim 4.

6. The host cell according to claim 5, wherein the cell is a human cell, preferably wherein the cell is selected from the group of patient's autologous cells.

7. A pharmaceutical composition or a diagnostic agent comprising as an agent at least one miRNA according to claim 1 or 2, the double-stranded nucleic acid of claim 3, the vector of claim 4 and/or the host cell of claim 5 or 6.

8. The pharmaceutical composition or diagnostic agent of claim 7, wherein the active agent is embedded in artificial exosomes.

9. The miRNA of claim 1 or 2, the pharmaceutical composition of claim 7 or 8 or the diagnostic agent of claim 7 or 8 for use in treatment or diagnosis of a pregnancy-associated disease.

10. The miRNA of claim 1 or 2, the pharmaceutical composition of claim 7 or 8 or the diagnostic agent of claim 7 or 8 according to claim 9, wherein the pregnancy-associated disease is selected from the group of diseases consisting of eclampsia, pre-eclampsia, HELLP-syndrome and failure or problems of placentation or implantation.
